# EUROPEAN PATENT SPECIFICATION

(11) **EP 3 986 500 B1**
(45) Date of publication and mention of the grant of the patent: **30.04.2025**
(21) Application number: 20740338.7
(22) Date of filing: 20.07.2020
(51) Int. Cl.: A61M 1/36, A61M 1/38, A61M 1/34

(54) **REMOVAL OF TUMOR CELLS FROM INTRAOPERATIVE AUTOLOGOUS BLOOD SALVAGE BY USING A TRIFUNCTIONAL ANTIBODY**
ENTFERNUNG VON TUMORZELLEN AUS AUTOLOGEM BLUT GESAMMELT DURCH INTRAOPERATIVE AUTOTRANSFUSION MITTELS EINES TRIFUNKTIONALEN ANTIKÖRPERS
ÉLIMINATION DE CÉLLULLES TUMORALES DU SANG AUTOLOGUE OBTENU PAR RÉCUPÉRATION PÉRI-OPÉRATOIRE EN UTILISANT UN ANTICORPS TRIFONCTIONNEL

(30) Priority: 18.07.2019 EP 19186959
(43) Date of publication of application: 27.04.2022
(73) Proprietor: Lindis Blood Care GmbH, 16761 Henningsdorf (DE)
(72) Inventor: LINDHOFER, Horst, 80639 Munich (DE)
(74) Representative: Isarpatent
(86) International application number: PCT/EP2020/070459
(87) International publication number: WO 2021/009383

(56) References cited:
- WO-A1-2013/113615
- US-A1- 2014 369 985

## Description

### Field of the Invention

The invention relates to a method performed ex vivo for removal of tumor cells from intraoperatively collected blood salvage by using a trifunctional bispecific antibody, and to said antibody for use in a method for removal of tumor cells from intraoperatively collected blood salvage.

### Background of the Invention

Since the AIDS epidemic of the early 1980s the interest in alternatives to allogeneic blood transfusion has grown, particularly for elective surgery. One alternative that currently accounts for over 5 % of the blood donated in the United States and some countries in Europe is autologous transfusion, obtained primarily by preoperative donation. In addition to preoperative blood donation, intraoperative blood salvage (IBS) (Table 1) from the surgical field represents an important option for covering transfusion demands. Along this IBS process, blood lost by a surgical patient is collected, cleaned, and made available for reinfusion to that patient.

Briefly, blood shed into the surgical field is aspirated from this site into an especially designed containment. Citrate or heparin anticoagulant is added, and the contents are centrifuged and/or filtered to remove leukocytes and clots and debris. IBS devices used can vary from simple, inexpensive, sterile bottles filled with anticoagulant to expensive, sophisticated, high speed cell washing devices (e.g. Medtronic Sequestra 1000, Cobe BRAT 2, Medtronic Autolog, Haemonetics Cell Saver-5^{®} and Fresenius CATS^{®}; Bentzien et al., Anaesthesist 49: 505, 2000; Serrick et al., J. Extra Corpor. Technol. 35(1): 28, 2003; Carless et al., The Cochrane Review, In: The Cochrane Library, John Wiley & Sons, Ltd., issue 3 pp. 1-180, 2010). Used in close to a million surgeries each year in the USA, the IBS procedure has become an integral part of blood management and conservation programs of hospitals.

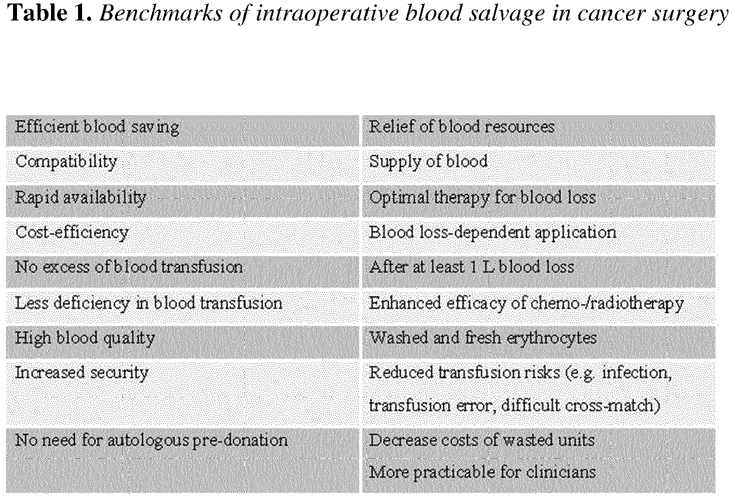

Besides increased incidences of preoperative anemia, blood donation prior surgery raises serious additional economical questions due to the high overall transfusion rates (Carless et al., Transfus. Med. 14: 123, 2004). In addition, about 30 % of preoperative autologous blood donations have to be discarded in Italy because not-required autologous blood products are excluded from allogeneic blood transfusions *per se* according to regulatory guidelines. In this regard IBS and subsequent autotransfusion generally represent safe and more cost-effective measures in blood management.

### Advantages of intraoperative blood salvage

In contrast to allogeneic red blood cell transfusions, IBS is considered a safe and efficacious alternative. Importantly, despite excellent viral diagnostics the risk of transferring HIV infection is 1 per 493.000 allogeneic blood transfusions, the risk of transferring hepatitis C virus infection is 1 per 103.000 and the risk of transmitting hepatitis B virus infection is 1 per 63.000 (Schreiber et al., N. Engl. J. Med. 334: 1685, 1996). Results of a 'Serious Hazards of Transfusions' study performed from 1996 until 2001 documented these risks of allogeneic transfusion incidences (Dzik et al., Transfusion 43: 1190, 2003). Strikingly, the transmission of infectious diseases by means of contaminated blood transfusions appears to be a minor risk compared to the enormous risks of ABO-incompatible blood due to administrative or human failure along the blood donation and transfusion process (Sazama, Transfusion 30: 583, 1990; Dzik et al., Transfusion 43: 1190, 2003). More than 70 % of transfusion incidences could be attributed to incorrect transfused blood components mainly caused by administration failure and errors in sampling, prescription as well as in component collection (Dzik et al., Transfusion 43: 1190, 2003). Pre- and intraoperative autotransfusion of red blood cells bypasses *per se* these inherent risks of allogeneic blood transfusion. In general, autologous blood salvage techniques offer advantages but do not require infusions of crystalloid or colloid to preserve blood volume (Table 1). Many liters of blood can be salvaged intraoperatively during extensive bleeding, far more than with other autologous techniques.

### Suitability of patients for IBS

Intraoperative blood salvage has been available for over 25 years. It is used extensively in cardiothoriac surgery, vascular and trauma surgery, as well as liver transplantation. Contraindications to its use are bacterial infections and tumor cells probably shed into the blood of the surgery field, and use of microfibrillar collagen or other foreign material at the operative site. Nowadays, due to shortages of donor blood and fears of transmitted infections the use of IBS also gains great interest in cancer surgery with high blood loss. The reluctance of surgeons to use autotransfusion in cancer surgery has diminished, as reports found no increase in local recurrence or metastatic disease when compared with standard survival data (Klimberg et al., Arch. Surg. 212: 1326, 1986; Perseghin et al., Vox Sang. 72: 221, 1997). As reviewed by Vanderlinde et al. (BMJ 324: 772, 2002) red blood cell autotransfusions collected from preoperative blood donations could significantly lead to reduced infection and recurrence incidences during colorectal cancer surgery as compared to allogeneic transfusions (Table 2). Some of these reviewed clinical trials are even more important as colorectal surgeries have been *per se* excluded from IBS recommendations due to the inherent risk of transmitting bacterial infections.

**Table 2. Clinical outcome of randomized trials of autologous versus allogeneic transfusion***

| **Study** | **No of patients** | **Type of surgery** | **Intervention** | **Type of complication** | **% of cases developing complications after transfusion** | | **P value for reduction in postoperative complications** |
|---|---|---|---|---|---|---|---|
| | | | | | **Autologous** | **Allogeneic** | |
| Busch et al, 1993⁹ | 423 | Colorectal | Predeposit autologous donation | Infection | 27 | 25 | NS |
| N. Engl. J. Med. 328: 1372, 1993 | | | | Recurrence | 37 | 34 | NS |
| Heiss et al, 1993 and 1994^{10, 14} | 120 | Colorectal | Predeposit autologous donation | Infection | 12 | 27 | <0.05 |
| Lancet 342: 1328, 1993 | | | | | | | |
| J. Clin. Oncol. 12: 1859, 1994 | | | | Recurrence | 17 | 29 | 0.11 |
| Newman et al, 1997¹¹ | 70 | Knee replacement | Postoperative autologous salvage | Infection | 6 | 34 | <0.05 |
| J. Bone Joint Surg. Br. 79: 630, 1997 | | | | | | | |
| Farrer et al, 1997¹² | 50 | Vascular | Intraoperative autologous salvage | Infection | 13 | 44 | 0.029 |
| J. Vasc. Nurs. 15: 111, 1997 | | | | | | | |
| Thomas et al, 2001¹³ | 231 | Knee replacement | Postoperative autologous salvage | Infection | NA | NA | 0.036 |
| Br. J. Anaesth 86: 669, 2001 | | | | Readmission | NA | NA | 0.008 |

| | | | | | | | |
|---|---|---|---|---|---|---|---|
| NS=not significant. NA=not available (ie not reported). * Table is taken from Vanderlinde et al., BMJ 324: 772, 2002. | | | | | | | |

Nevertheless, a study clearly showed that tumor cells were detectable in the surgery field, although their impact on cancer recurrence remained unclear (Hansen et al., Arch. Surg. 130: 387, 1995). For instance the number of tumor cells in the peripheral blood and IBS of 61 patients with cancer surgery of an abdominal, orthopedic, urological, gynecological, or head and neck malignant tumor were compared. In 57 of 61 patients tumor cells were detected in the blood shed during oncological surgery. These tumor cells were identified by proliferation capacity, invasiveness, and tumorigenicity with a sensitivity of 10 tumor cells per 500 mL blood (Hansen et al., Arch. Surg. 130: 387, 1995). Interestingly, the number of tumor cells in the shed blood did not correlate with the amount of blood loss and only in 26 % of these patients circulating tumor cells could be detected in the peripheral blood. Therefore, it is estimated that the number of tumor cells in the blood shed into the surgery field could range from 10 to 10⁷. These results were also confirmed independently by Dale et al., Br. J. Surg. 75: 581, 1988 and Müller et al., Anaesthesist 45: 834, 1996.

To further address safety concerns about the risk of residual tumor cells in IBS samples, additional approaches are needed to effectively eliminate the contaminating tumor cells:
- One method used in combination with automatic IBS devices like Cell Saver-5^{®} is represented by additional filtration of samples through leukocytes depletion filters (e.g. Pall RC400, RCEZ1T), RC XL-1) (Bontadini et al., Transfusion 34. 531, 1994; Yaprak et al., Turk. J. Pediatr. 40: 89, 1998; Gwak et al., Liver Transplant. 11: 331, 2005). The safe transfusion of erythrocytes collected by IBS does not impair clinical outcome as evaluated in several trials (Edelman et al. Urology 47 : 179, 1996; Perseghin et al., Vox Sang. 72: 221, 1996; Davis et al., BJU International 91: 474, 2003).
- In a different IBS approach the samples are irradiated at 50 Gy due to the underlying principle of radiosensitivity of nucleated cancer cells and due to the radioresistance of non-nucleated red blood cells.

Due to the complex logistic demand of this IBS/irradiation approach including additional staff requirement, dosimetry issues, suitable as well as certified irradiation equipment in the clinical department, this latter procedure is not in favor of broad application. In contrast, the filtration procedure for leukocyte depletion represents an elegant approach to reduce residual tumor cells during IBS although this technique still has the inherent risk of residual tumor cells still passing the filter.

Edmund A.M. Neugebauer: "Jahresbericht 2010, Institut für Forschung in der Operativen Medizin", Universität Witten / Herdecke, 1 March 2011 (2011-03-01), page 1-98 discloses trifunctional antibody Catumaxomab for elimination of free tumor cells from intra-operatively collected blood of wounds for auto transfusion during oncologic surgery.

Alexandra Schoberth: "Entwicklung und Verbesserung von Methoden zur Charakterisierungdisseminierter Tumorzellen bei soliden Tumoren", Dissertation an der Medizinischen Fakultät der Ludwig-Maximilians-Universität zu München, 9 December 2004 (2004-12-09) discloses the removal (purging of tumor cells from autologous stem cell apharesis products (for readministration) ex vivo using trifunctional antibodies.

Schoberth A et al: "A new class of trifunctional bispecific antibodies mediated efficient immunological purging of peripheral blood stem cells", European Journal of Cancer, vol. 37, 1 September 2001 (2001-09-01), page S51 describes that trifunctional bispecific antibodies may be efficiently be used for immunological purging of peripheral blood stem cells.

US2014/369985 describes an ex vivo method for the removal of tumor cells from intraoperatively salvaged blood using trifunctional bispecific antibodies which bind to a T cell, to a tumor-associated antigen on a tumor cell and, via its Fc-portion, to an Fc-receptor positive cell. However, binding to T cells, particularly to CD3 and CD28 as T cell surface markers, implies a certain risk of a cytokine release, and said cytokines might enter the erythrocyte concentrate collected in order to be re-infused into the patient. Reinfusion of said erythrocyte concentrate potentially comprising cytokines might cause severe inflammation reactions in the recipient. Further, the method might not sufficiently remove tumor cells in patients with a reduced number of T cells.

### Problems to be solved by the present Invention

Therefore, for further enhanced safety in cancer surgery to remove residual tumor cells, all known methods for reintroducing autologous blood obtained e.g. during surgery from wounds of patients with tumors have to be improved in order to provide a reliable removal of possibly contaminating tumor cells; said improvement should also include a high efficiency of removal of the tumor cells from the autologous blood of patients; additionally, said improvement should be capable of being easily implemented in e.g. devices like Cell Saver-5^{®} or CATS^{®} or into any other methods to reintroduce blood free or at least substantially free of tumor cells from patients who had undergone surgery, wherein preferably the occurrence of said effects is significantly decreased.

### Summary of the Invention

The novel approach found by the present inventors is based on antibody-mediated formation of multi-cellular complexes, i.e. associates and/or aggregates, comprising tumor cells recognized by antibody-driven tumor-associated antigen recognition, and further by recognition of -leukocytes and/or Fc-receptor positive accessory cells and/or further tumor cells followed by removal of said associates by e.g. centrifugation and/or filtration steps.

### Detailed Description of the Invention and Preferred Embodiments

The following discussion is included for purposes of describing the present invention and illustrating preferred embodiments thereof.

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of the present disclosure, suitable methods and materials are described below. The term "comprises" means "includes." All publications, patent applications, patents, and other references mentioned herein are incorporated by reference in their entirety. In case of conflict, the present specification, including explanations of terms, will control. In addition, the materials, methods, and examples are illustrative only and not intended to be limiting.

The present invention concerns:
(1) an ex vivo-method for removal of tumor cells from intra-operatively obtained blood salvage, comprising the following steps:
   - providing intra-operatively salvaged blood which may contain tumor cells;
   - contacting said intra-operatively salvaged blood with at least one trifunctional bispecific antibody
      wherein said at least one antibody comprises the following properties:
         a) binding to a pan-leukocyte antigen, which is selected from at least one member of the group consisting of CD11a, CD15, CD18, CD29, CD39, CD45, CD48, CD52, CD55, CD58, CD59, CD82, CD95, CD97, CD122, CD124, CD132 and CDw137;
         b) binding to a tumor-associated antigen on a tumor cell;
         c) binding via its Fc-portion to an Fc-receptor positive cell,
      said at least one antibody being capable of forming a 3-dimensional network at least with tumor cells and leukocyte cells contained in the blood,
      wherein said at least one antibody is contacted with said intra-operatively salvaged blood for a time period of 10 - 240 minutes, preferably 10 - 180 minutes, which is sufficient to cross-link tumor cells comprising said tumor-associated antigen and leukocyte cells comprising said pan-leukocyte antigen, and/or other tumor cells in order to obtain associates and/or aggregates comprising said antibody,
      wherein said tumor cells, said other tumor cells and said leukocyte cells are potentially present in the intra-operatively salvaged blood, and wherein said tumor cells are specifically recognized by said at least one antibody; and

      - mechanically removing said associates and/or aggregates from said intra-operatively salvaged blood.
(2) Preferably, said trifunctional bispecific antibody is selected of a group of antibodies with the following isotype combinations:
   rat-IgG2b/mouse-IgG2a,
   rat-IgG2b/mouse-IgG2b,
   rat-IgG2b/human-IgG1,
   mouse-[VH-CH1; VL-CL]-human-IgG1/rat-[VH-CH1, VL-CL]-human-IgG1-[hinge]-human-IgG3*-[CH2-CH3]
   [* = Caucasian allotypes G3m(b+g) = no binding to protein A].
(3) Preferably, in the ex vivo method said pan-leukocyte antigen is CD52.
(4) Preferably, in the ex vivo method at least two different antibodies are applied which tumor antigen specificity and/or pan-leukocyte antigen specificity differ from each other.
(5) Preferably, in the ex vivo method said associates are comprised of antibodies, tumor cells and leukocyte cells.
(6) Preferably, in the ex vivo method said one or more antibodies and/or scaffold proteins are applied in an amount of 1µg to 20µg, preferably 1 to 10µg or 1 to 5µg or 1 to 2µg per liter of intraoperatively salvaged blood.
(7) Preferably, the ex vivo method comprises at least one of the following further steps:
   a) mixing of the intraoperatively salvaged blood before addition of said at least one antibody with at least one anticoagulating agent;
   b) separating erythrocytes from said associates and/or aggregates and further blood components via centrifugation, preferably by density gradient centrifugation;
   c) optionally filtration of said mixture
      to remove potentially residual associates and/or aggregates and residual cell complexes; and
   d) collecting the erythrocyte-containing fraction and further blood components in separate containers.
(8) Preferably, in the ex vivo method the incubation time of said at least one antibody with said intraoperatively salvaged blood is between 10 and 90 minutes, further preferably between 20 and 60 minutes, optionally wherein said incubation is performed at a temperature of between 19 to 25°C, preferably at room temperature.
(9) Preferably, in the ex vivo method , said removal of associates and/or aggregates is by centrifugation, filtration or a combination thereof.
(10) Preferably, in the ex vivo method leukocytes and/or tumor cell containing cell complexes are removed in a separate step using a leukocyte adsorption filter.
(11) Preferably, in the ex vivo method said tumor cells are from epithelial, hematological or neuroectodermal tumors.
(12) Preferably, in the ex vivo method , wherein additionally trifunctional bispecific antibodies are administered which bind to a T cell, to a tumor-associated antigen on a tumor cell, and via its Fc-portion to an Fc-receptor positive cell.
(13) Preferably, in the ex vivo method said at least one antibody and/or said at least one scaffold protein comprises a property of binding to two different tumor-associated antigens. Said two different tumor associated antigens can be located on two different tumor cells respectively, or can be located on the same tumor cell.
(14) Use of a bispecific trifunctional antibody, with the following properties:
   a) binding to a pan-leukocyte antigen, which is selected from at least one member of the group consisting of CD11a, CD15, CD18, CD29, CD39, CD45, CD48, CD52, CD55, CD58, CD59, CD82, CD95, CD97, CD122, CD124, CD132 and CDw137;
   b) binding to a tumor-associated antigen on a tumor cell;
   c) binding via its Fc-portion to an Fc-receptor positive cell,

   said antibody being capable of forming a 3-dimensional network with tumor cells and leukocyte cells contained in the blood, in an ex vivo method according to one or more of (1) to (13), wherein preferably
   said at least one antibody comprises a property of binding to two different tumor-associated antigens. Said two different tumor associated antigens can be located on two different tumor cells respectively, or can be located on the same tumor cell.
(15) A trifunctional bispecific antibody for use in a method for treating cancer, wherein the method comprises the following steps:
   - providing intra-operatively salvaged blood which may contain tumor cells;
   - contacting said intra-operatively salvaged blood with said antibody, wherein said antibody comprises the following properties:
      a) binding to a pan-leukocyte antigen, which is selected from at least one member of the group consisting of CD11a, CD15, CD18, CD29, CD39, CD45, CD48, CD52, CD55, CD58, CD59, CD82, CD95, CD97, CD122, CD124, CD132 and CDw137;
      b) binding to a tumor-associated antigen on a tumor cell;
      c) binding via its Fc-portion to an Fc-receptor positive cell,

      said antibody being capable of forming a 3-dimensional network at least with tumor cells and leukocyte cells contained in the blood,
      wherein said antibody is contacted with said intra-operatively salvaged blood for a time period of 10 - 240 minutes, preferably 10 - 180 minutes, which is sufficient to cross-link tumor cells comprising said tumor-associated antigen and leukocyte cells comprising said pan-leukocyte antigen, and/or other tumor cells in order to obtain associates and/or aggregates comprising said antibody, wherein said tumor cells, said other tumor cells and said leukocyte cells are potentially present in the intra-operatively salvaged blood, and wherein said tumor cells are specifically recognized by said antibody; and

      - mechanically removing said associates and/or aggregates from said intra-operatively salvaged blood.
(18) Preferably, the antibody for use according to (17) comprises a property of binding to two different tumor-associated antigens. Said two different tumor associated antigens can be located on two different tumor cells respectively, or can be located on the same tumor cell.

Other preferred embodiments are described in the following description and the examples in combination with the figures. The scope of the invention is defined by the appended claims.

The presently described method is practiced *ex vivo,* i.e. outside the human body. Intra-operative blood salvage (IBS) is well known in the art and is also described as "autologous blood salvage". Blood which is lost during surgery is recovered and re-infused into the same patient from whom the blood lost during surgery has been gained.

The method for removal of tumor cells from intra-operatively salvaged blood comprises the steps as described herein for the ex vivo method and what was said with regard to the ex vivo method applies accordingly to this method, where appropriate.

The presently described method is directed to the removal of tumor cells which potentially might contaminate intra-operatively obtained blood salvage. The blood is preferably obtained from patients who undergo a surgery treatment and suffer from a tumor and/or a cancer or who are suspected to harbor cells which might be considered to be tumorigenic.

Preferably, although not part of the invention, the method starts with the surgeon aspirating blood from the surgical field. The collecting step is preferably performed via a suction device and dilutives like e.g. sterofundin can also be used during this step. Other dilutives such as 0.9% NaCl with Heparin can also be used. The aspirated blood can be then mixed with an anticoagulant in order to avoid coagulation of the blood. The aspirated blood may be collected in a reservoir until there is sufficient blood for processing.

Preferably, aspirated blood is diluted with dilutives like e.g. sterofundin and/or 0.9% NaCl with Heparin.

Reference to an "anti-coagulant" includes classes of anti-coagulants such as anticoagulants, anti-platelet drugs, thrombolytic and fibrinolytic drugs and metal ion chelators agents such as e.g. citrate, citrate dextrose (ACD) and EDTA and oxalate. In a related aspect, the anti-coagulants include heparin and glycosaminoglycans such as low molecular weight heparin such as Bemiparin, Certoparin, Dalteparin, Enoxaparin, Nadroparin, Pamaparin, Reviparin and Tinzaparin and heparinoid such as Danaparoid, Sulodexide, Dermatan sulfate; direct thrombin (II) inhibitors such as Argatroban, Bivalirudin, Dabigatran, Desirudin, Hirudin, Lepirudin, Melagatran, Ximelagatran; Fact. Xa inhibitors (such as Tick Anticoagulant Peptide), such as Apixaban, Otamixaban, Rivaroxaban and oligosaccharides such as Fondaparinux and Idraparinux; Vitamin K antagonists such as Acenocoumarol, Clorindione, Coumatetralyl, Dicoumarol (Dicumarol), Diphenadione, Ethyl biscoumacetate, Phenprocoumon, Phenindione, Tioclomarol and Warfarin.

In one embodiment of the present invention, the so collected blood salvage might be tested on the presence of tumor cells wherein particularly preferred the type of tumor-associated antigen is determined by well-known methods in the art, e.g. by labeled antibodies specifically reacting with epitopes of tumor-associated antigens in order to determine the type tumor-antigen to which said antibody binds and which is to be applied in the invention.

In the present invention, the term "tumor cell" refers to any cell which can divide relentlessly and form solid tumors or flood the blood with abnormal cells. Preferably, in the present invention, the tumor cells present in the intro-operatively salvaged blood can be from epithelial, haematological or neuroectodermal tumors.

Examples of tumors included in the present invention (but not limited thereto) comprise sarcomas and carcinomas, including fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer (including basal breast carcinoma, ductal carcinoma and lobular breast carcinoma), lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, astrocytoma, medulloblastoma, craniopharyrgioma, ependymoma, pinealoma, hemangioblastoma, acoustic neuroma, oligodendroglioma, meningioma, melanoma, neuroblastoma and retinoblastoma). Further examples include epithelial tumors, hematological tumor and neuroectodermal tumors.

Preferably, in the method of the present invention, said tumor cells are from epithelial, hematological or neuroectodermal tumors.

In the present invention, the "immune cells" include leukocyte and Fc-receptor positive cells. In the present invention, the "Fc-receptor cell" refers to cells with Fc-receptor present on the cell surface. Preferably, the "Fc-receptor positive cell" refer to one or more of monocyte, macrophage, dendritic cell, natural killer cell, neutrophil and eosinophilic cell.

Preferably, the intra-operatively salvaged blood is collected in a reservoir, before contacting with said antibody or said scaffold protein.

The intra-operative blood salvage is then contacted with an antibody or an appropriate protein scaffold which is capable of specifically binding to at least one epitope of at least one tumor-associated antigen of at least one tumor cell.

In the present invention, "erythrocyte concentrate" refers to packed red cells.

In the present invention, "cell aggregate(s)" refers to cells which are adhered to each other, and said expression is interchangeable with "associate(s)" or "cell associate(s)". Similarly, "associating" and "aggregating" are also interchangeable.

As a further requirement, said antibody must be capable of forming a 3-dimensional network of at least said antibodies and said tumor cells contained in the blood in order to obtain aggregates or associates comprising said antibodies and tumor cells. In a further particularly preferred embodiment of the present invention, said antibody is also capable of binding to more than one tumor cells and/or to immune cells in order to form a three-dimensional network of antibodies and tumor cells and optionally immune cells and further optionally additional tumor cells. Due to the composition, structure and size of said associates resulting from the formation of multi-cellular complexes, the associates are capable of being removed from said IBS by centrifugation or filtration or a combination thereof. It is to be understood that filtration and centrifugation are preferred methods while other methods in order to efficiently remove said associates comprising residual tumor cells might be recognized by the person skilled in the art.

The invention therefore preferably focuses on the mechanical removal of associates and aggregates formed by associating and aggregating antibodies with tumor-associated antigens on tumor cells and optionally immune cells and/or other tumor cells and not on the depletion of tumor cells carrying tumor-associated antigens by destruction of said tumor cells by antibody-specific interactions and immunological effects. While using antibodies in a conventional way as immunological agents in order to destroy tumor cells by interacting with immune cells, the invention gains benefit preferably on the antibodies' capacity of being capable in crosslinking antigens which are in the present case located on tumor cells or fragments thereof. The effects of the invention are achieved by the interaction of the antibodies with said tumor cells and particularly by removal of said three-dimensional network by centrifugal and/or filtration separation methods.

The present method can be also used in combination with other methods known in the art wherein antibodies or antibody-like molecules may be linked to magnetic components like magnetic beads or other structural elements which facilitate removal of antibody complexes via said structural elements, or tumor cells are removed by cell sorting methods like flow cytometry. Only due to the formation of a 3-dimensional network of antibodies with antigens like tumor-associated antigens on tumor cells or fragments thereof, and optionally immune cells and/or other tumor cells, a mechanical removal of said associates is possible. In this regard, in the present invention it is not necessary to involve the other structural components like magnetic beads or fluorescent molecules to facilitate said removal, which renders the present method more simple and easier to perform.

The present invention is herein described and claimed with respect to trifunctional bispecific antibodies within the limitations of the present invention, and exemplified with respect to the trifunctional bispecific antibody anti-CD52 x anti-EpCAM. Said anti-CD52 x anti-EpCAM trifunctional bispecific antibody is directed against tumor-associated antigen EpCAM, and is additionally binding to CD52, a pan-leukocyte marker and to Fc-receptor positive cells by its Fc-portion. The specific embodiments described in the examples have to be understood as exemplary embodiments which provide evidence for the feasibility of the present invention. Having provided evidence on the excellent removal of tumor cells from IBS by Catumaxomab, proof of concept has been given for the principle the presently claimed method is based upon. Having provided this evidence, he person skilled in the art will inevitably have the possibility to expand the concept to other tumors and other antibodies as far as covered by the present invention which are able to interact with said tumor cells and optionally said immune cells in order to provide for a three-dimensional network, i.e. multi-cellular complexes which can be removed for instance by centrifugation and/or filtration.

Preferably, the antibodies of the present invention are specifically selected from trifunctional antibodies. Preferably, the trifunctional antibody in the present invention can be bi-, tri-, tetra- and multispecific antibodies. The trifunctional antibody disclosed above refers to trifunctional bispecific antibody. However, if the tri-, tetra- and multispecific antibodies also exhibit the same properties or effects, said trifunctional antibody can also refer to a trifunctional tri-, tetra- and multispecific antibody as used herein.

The antibodies of the present invention are specifically selected from trifunctional antibodies. A bispecific antibody is defined as an antibody capable of binding to two different types of antigens preferably via its variable region; a trispecific antibody is characterized by binding to three different types of antigens preferably via its variable region; a tetraspecific antibody is characterized by binding to four different types of antigens preferably via its variable region while a multispecific antibody is defined as being capable of binding multiple different types of antigens preferably via its variable region. As one specific example, the trifunctional bispecific antibody anti-CD52 x anti-EpCAM is defined by binding to the tumor-associated antigen EpCAM on the one hand and to the pan-leukocyte marker CD52 on the other hand as well as with accessory cells by its Fc part.

The bi-, tri-, tetra- and multispecific antibodies described above may be monovalent, divalent, trivalent, tetravalent or multivalent. An antibody with a monovalent binding property is defined as an antibody which is capable of binding to one tumor-associated antigen. A bivalent monoclonal antibody is defined as an antibody which is capable of binding to two tumor-associated antigens or one tumor-associated antigen and one immune cell-associated antigen. A trivalent monoclonal antibody is defined as an antibody which is capable of binding to three different tumor-associated antigens or two tumor-associated antigens and one immune cell-associated antigen or one tumor-associated antigen and two immune cell-associated antigens. A tetravalent monoclonal antibody is defined as an antibody which is capable of binding to four different tumor-associated antigens or two different tumor-associated antigens - each having two identical antigen binding arms - or two/three tumor-associated antigens and one immune cell-associated antigen or two tumor-associated antigens and two immune cell-associated antigens. A multivalent monoclonal antibody is defined as an antibody which is capable of binding to one or more tumor-associated antigens and/or one or more immune cell associated antigen. The term "binding to a tumor-associated antigen" is defined as binding to an epitope of said tumor-associated antigen on a tumor cell. Only those antibodies having the trifunctional bispecific format as described by claim 1 are covered by the invention. All other antibodies are described only for information purposes.

General description of bifunctional or trifunctional antibodies are described by Kontermann RE (ed.), Springer Heidelberg Dordrecht London New York, pp. 1-28 (2011) having bispecific or trispecific (trifunctional formats with bivalent, trivalent and tetravalent binding properties to one tumor-associated antigen and to one or more surface antigens of leukocytes (i.e. cells of the immune system) are of importance for this patent application.
- Bispecific antibody formats with bivalent antigen binding features:
   e.g. scFv (e.g. BiTE class), Db, scDb, dsDb, DART, dAb₂/VHH₂, knob-into-holes derivates, SEED-IgG, heteroFc-scfv, Fab-scFv, CrossMabs
- Bi- (tri-) specific antibody formats with trivalent antigen binding features:
   e.g. triple body, DNL-F(ab)₃, scFv_{2-CH1/CL}, dAb₃, Fab-scFv₂, IgG-scFab
- Bi- (tri-) specific antibody formats with tetravalent antigen binding features:
   e.g. IgG-scFv, scFv-IgG, scFv-Fc, F(ab')₂-scFv₂, sDb-Fc, scDb-C_{H}3, Db-Fc, scFv₂-H/L, DVD-Ig, tandAb, scFv-dhlx-scFv, dAb₂-IgG, two-in-one mAb, mAb², dAb-IgG, dAb-Fc-dAb.

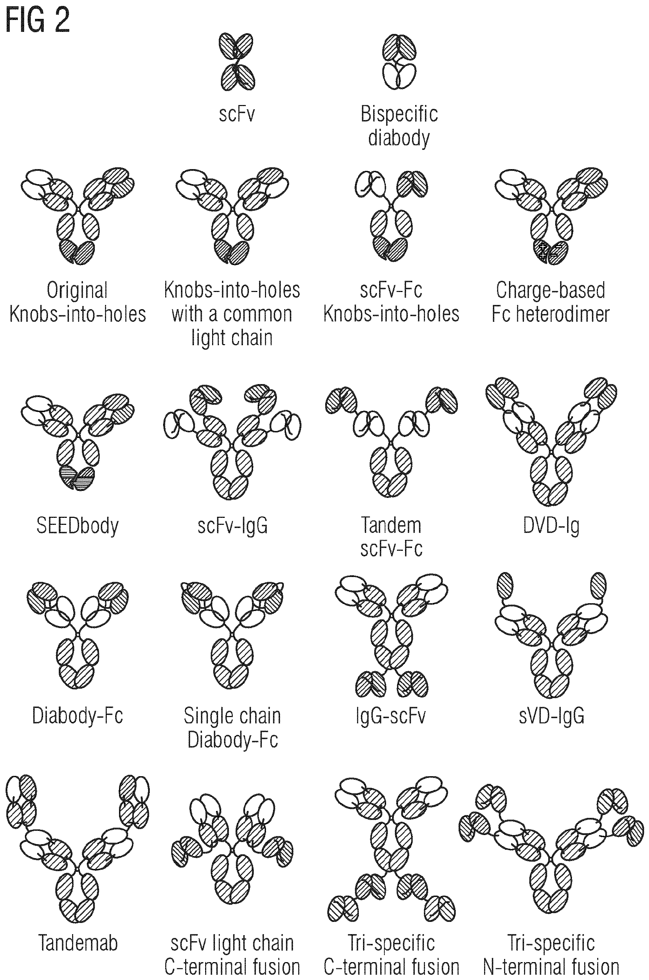

Further examples of antibodies to be used according to the invention are shown in the enclosed Figure 2.

Additional antibodies to be used according to the invention are described in the following references:
Müller D and RE Kontermann. In: Bispecific Antibodies. Kontermann RE (ed.), Springer Heidelberg Dordrecht London New York, pp. 83-100 (2011)
scFv (BiTE)
Baeuerle PA, Zugmaier G and D Rüttinger. In: Bispecific Antibodies. Kontermann RE (ed.), Springer Heidelberg Dordrecht London New York, pp. 273-288 (2011)

### DVD-Ig

Tarcsa E, Fraunhofer W, Ghayur T, Salfeld J and J Gu. In: Bispecific Antibodies. Kontermann RE (ed.), Springer Heidelberg Dordrecht London New York, pp. 171-186 (2011)

### DNL-derivatives

Chang C-H, Rossi EA, Sharkey RM, DM Goldenberg. In: Bispecific Antibodies. Kontermann RE (ed.), Springer Heidelberg Dordrecht London New York, pp. 199-216 (2011)

### Two-in-one antibodies

Koeing P and G Fuh. In: Bispecific Antibodies. Kontermann RE (ed.), Springer Heidelberg Dordrecht London New York, pp. 187-198 (2011)

### CrossMabs

Schaefer et al. Proc. Natl. Acad. Sci. USA 108: 11187 (2011)

Preferably, two or more trifunctional antibodies with different specificities can be combined for mediating the formation of said associates.

The trifunctional bispecific antibody used in the present invention is directed against a pan-leukocyte antigen, which is selected from a group consisting of CD11a, CD15, CD18, CD29, CD39, CD45, CD48, CD52, CD55, CD58, CD59, CD82, CD95, CD97, CD122, CD124, CD132 and CDw137.

Preferably, the trifunctional bispecific antibodies used in the present invention is directed against CD11a, CD18, CD45, CD52, CD82 or CD97. In a further preferred embodiment, the antibody is directed against CD52.

In a preferred embodiment, the antibodies used in the present invention are monoclonal antibodies. This is specifically true for the trifunctional bispecific antibodies described herein in detail.

Proteins having relatively defined three-dimensional structures are commonly referred to as protein scaffolds. These protein scaffolds may be used as reagents for the design of artificially engineered antibodies. These scaffolds typically contain one or more regions which are amenable to specific or random sequence variation, and such sequence randomization is often carried out to produce libraries of proteins from which the desired antibody scaffolds may be selected. Such scaffolds are particularly useful in the field of antibody design.

These antibody scaffolds are non-immunoglobulin proteins which mimic properties of a monoclonal antibody with respect to its binding activity to for instance tumor cells and immune cells. Scaffolds often include loops or domains which form the binding side of said antibody scaffold. These antibody mimics may be utilized for the purpose of designing proteins which are capable of binding to virtually any compound of interest. This directed evolution approach results in the production of antibody-like molecules with high affinities for antigens of interest. In addition, those scaffolds may be used to display defined exposed loops (e.g. loops previously randomized and selected on the basis of antigen binding) in order to direct evolution of molecules that bind to such introduced loops. Methods on how to obtain antibody-like scaffold proteins are known in the art. The following describes one possible approach for obtaining an antibody-like scaffold protein.

A first screening method, useful for the isolation or identification of randomized or mutated proteins of interest, involves: (a) contacting a compound of interest with a candidate protein, the candidate protein being a derivative non-antibody protein including a domain having an immunoglobulin-like fold, the non-antibody protein deriving from a reference protein by having a mutated amino acid sequence wherein the non-antibody protein binds with a Kd at least as tight as 1 microM to a compound that is not bound as tightly by the reference protein, wherein the contacting is carried out under conditions that allow compound-protein complex formation; and (b) obtaining, from the complex, the derivative protein that binds to the compound.

The second screening method is for isolating or identifying a compound which binds to a tumor-associated protein of interest. This method begins with a non-antibody protein including a domain having an immunoglobulin-like fold and deriving from a reference protein by having a mutated amino acid sequence, wherein the non-antibody protein binds with a Kd at least as tight as 1 microM to a compound that is not bound as tightly by the reference protein. This derivative protein is then contacted with a candidate compound (tumor-associated antigen or an epitope thereof), wherein the contacting is carried out under conditions that allow compound-protein complex formation, and the compound which binds to the derivative protein is obtained from the complex. Again, this general technique may be carried out with any protein.

Further methods of obtaining non-antibody proteins which bind to compounds of interest (tumor-associated antigen or an epitope thereof) are described as follows. One such method involves: (a) providing a non-antibody scaffold protein including an immunoglobulin-like fold, wherein the scaffold protein does not bind to the compound with a Kd as tight as 1 micro M; (b) generating mutated derivatives of the non-antibody scaffold protein, thereby producing a library of mutated proteins; (c) contacting the library with the compound; (d) selecting from the library at least one derivative protein which binds to the compound with a Kd at least as tight as 1 microM; and (e) optionally repeating steps (b)- (d) substituting for the non-antibody scaffold protein in repeated step (b) the product from the previous step (d). Again, this general technique may be carried out with any protein.

The so produced scaffold proteins mimic the function of an antibody as described above and below and can be used either instead of an immunoglobulin-based antibody or in combination with it. In the present invention, said trifunctional antibody can be replaced with said scaffold protein insofar both display the same function in respect of binding to a leukocyte, binding to a tumor-associated antigen on a tumor cell, and binding via its Fc-portion to an Fc-receptor positive cell, to obtain an intra-operatively salvaged blood containing cell aggregates.

In the present invention, said trifunctional antibody and said scaffold protein can be applied alone or in combination.

The following describes in more detail trifunctional bispecific and trispecific antibodies.

Preferably, the antibody used according to the invention comprises a binding site in its Fc-portion for Fc Υ (Fc gamma) receptor type I, II and/or III.

Preferably, the antibody used according to the invention is able to bind to monocytes, macrophages, dendritic cells, natural killer cells, neutrophils and/or eosinophilic cells being Fcγ receptor positive cells.

In the present invention, the tumor associated antigen refers to an antigenic substance produced in tumor cells.

Preferably, said tumor associated antigen the antibody or scaffold protein, trifunctional bispecific antibody binds to is selected from the group consisting of: EpCAM, Her2neu, EGFR, CD30, CD20, CD22, MUC1, MUC1* with changed glycosylation pattern, PSMA, CD33, MCSP, cMet, EphA2, Endosialin, Carboanhydrase, IGF-1R, FAP-alpha, CD19, GD2, CEA, FR, proteoglycans, G250, GC182, GT468, GT512.

More preferably, said tumor associated antigen the antibody or scaffold protein, the trifunctional bispecific antibody of the present invention binds to is EpCAM, Her2/neu, MUC1, EGFR, EphA2, GD2 or CD20.

Preferably, the trifunctional bispecific antibody of the present invention is directed against one pan-leukocyte antigen and one tumor-associated antigen, wherein such two antigens combination is selected from a group consisting of EpCAM/CD11a, EpCAM/CD15, EpCAM/CD18, EpCAM/CD29, EpCAM/CD39, EpCAM/CD45, EpCAM/CD48, EpCAM/CD52, EpCAM/CD55, EpCAM/CD58, EpCAM/CD59, EpCAM/CD82, EpCAM/CD95, EpCAM/CD97, EpCAM/CD122, EpCAM/CD124, EpCAM/CD132, EpCAM/CDw137, Her2neu/CD11a, Her2neu/CD15, Her2neu/CD18, Her2neu/CD29, Her2neu/CD39, Her2neu/CD45, Her2neu/CD48, Her2neu/CD52, Her2neu/CD55, Her2neu/CD58, Her2neu/CD59, Her2neu/CD82, Her2neu/CD95, Her2neu/CD97, Her2neu/CD122, Her2neu/CD124, Her2neu/CD132, Her2neu/CDw137, EGFR/CD11a, EGFR/CD15, EGFR/CD18, EGFR/CD29, EGFR/CD39, EGFR/CD45, EGFR/CD48, EGFR/CD52, EGFR/CD55, EGFR/CD58, EGFR/CD59, EGFR/CD82, EGFR/CD95, EGFR/CD97, EGFR/CD122, EGFR/CD124, EGFR/CD132, EGFR/CDw137, CD30/CD11a, CD30/CD15, CD30/CD18, CD30/CD29, CD30/CD39, CD30/CD45, CD30/CD48, CD30/CD52, CD30/CD55, CD30/CD58, CD30/CD59, CD30/CD82, CD30/CD95, CD30/CD97, CD30/CD122, CD30/CD124, CD30/CD132, CD30/CDw137, CD20/CD11a, CD20/CD15, CD20/CD18, CD20/CD29, CD20/CD39, CD20/CD48, CD20/CD55, CD20/CD58, CD20/CD59, CD20/CD82, CD20/CD95, CD20/CD97, CD20/CD122, CD20/CD124, CD20/CD132, CD20/CDw137, CD22/CD11a, CD22/CD15, CD22/CD18, CD22/CD29, CD22/CD39, CD22/CD45, CD22/CD48, CD22/CD52, CD22/CD55, CD22/CD58, CD22/CD59, CD22/CD82, CD22/CD95, CD22/CD97, CD22/CD122, CD22/CD124, CD22/CD132, CD22/CDw137, MUC1/CD11a, MUC1/CD15, MUC1/CD18, MUC1/CD29, MUC1/CD39, MUC1/CD45, MUC1/CD48, MUC1/CD52, MUC1/CD55, MUC1/CD58, MUC1/CD59, MUC1/CD82, MUC1/CD95, MUC1/CD97, MUC1/CD122, MUC1/CD124, MUC1/CD132, MUC1/CDw137, MUC1*/CD11a, MUC1*/CD15, MUC1*/CD18, MUC1*/CD29, MUC1*/CD39, MUC1*/CD45, MUC1*/CD48, MUC1*/CD52, MUC1*/CD55, MUC1*/CD58, MUC1*/CD59, MUC1*/CD82, MUC1*/CD95, MUC1*/CD97, MUC1*/CD122, MUC1*/CD124, MUC1*/CD132, MUC1*/CDw137, PSMA/CD11a, PSMA/CD15, PSMA/CD18, PSMA/CD29, PSMA/CD39, PSMA/CD45, PSMA/CD48, PSMA/CD52, PSMA/CD55, PSMA/CD58, PSMA/CD59, PSMA/CD82, PSMA/CD95, PSMA/CD97, PSMA/CD122, PSMA/CD124, PSMA/CD132, PSMA/CDw137, CD33/CD11a, CD33/CD15, CD33/CD18, CD33/CD29, CD33/CD39, CD33/CD45, CD33/CD48, CD33/CD52, CD33/CD55, CD33/CD58, CD33/CD59, CD33/CD82, CD33/CD95, CD33/CD97, CD33/CD122, CD33/CD124, CD33/CD132, CD33/CDw137, MCSP/CD11a, MCSP/CD15, MCSP/CD18, MCSP/CD29, MCSP/CD39, MCSP/CD45, MCSP/CD48, MCSP/CD52, MCSP/CD55, MCSP/CD58, MCSP/CD59, MCSP/CD82, MCSP/CD95, MCSP/CD97, MCSP/CD122, MCSP/CD124, MCSP/CD132, MCSP/CDw137, cMet/CD11a, cMet/CD15, cMet/CD18, cMet/CD29, cMet/CD39, cMet/CD45, cMet/CD48, cMet/CD52, cMet/CD55, cMet/CD58, cMet/CD59, cMet/CD82, cMet/CD95, cMet/CD97, cMet/CD122, cMet/CD124, cMet/CD132, cMet/CDw137, EphA2/CD11a, EphA2/CD15, EphA2/CD18, EphA2/CD29, EphA2/CD39, EphA2/CD45, EphA2/CD48, EphA2/CD52, EphA2/CD55, EphA2/CD58, EphA2/CD59, EphA2/CD82, EphA2/CD95, EphA2/CD97, EphA2/CD122, EphA2/CD124, EphA2/CD132, EphA2/CDw137, Endosialin/CD11a, Endosialin/CD15, Endosialin/CD18, Endosialin/CD29, Endosialin/CD39, Endosialin/CD45, Endosialin/CD48, Endosialin/CD52, Endosialin/CD55, Endosialin/CD58, Endosialin/CD59, Endosialin/CD82, Endosialin/CD95, Endosialin/CD97, Endosialin/CD122, Endosialin/CD124, Endosialin/CD132, Endosialin/CDw137, Carboanhydrase /CD11a, Carboanhydrase /CD15, Carboanhydrase /CD18, Carboanhydrase /CD29, Carboanhydrase /CD39, Carboanhydrase /CD45, Carboanhydrase /CD48, Carboanhydrase /CD52, Carboanhydrase /CD55, Carboanhydrase /CD58, Carboanhydrase /CD59, Carboanhydrase /CD82, Carboanhydrase /CD95, Carboanhydrase /CD97, Carboanhydrase /CD122, Carboanhydrase /CD124, Carboanhydrase /CD132, Carboanhydrase /CDw137, IGF-1R/CD11a, IGF-1R/CD15, IGF-1R/CD18, IGF-1R/CD29, IGF-1R/CD39, IGF-1R/CD45, IGF-1R/CD48, IGF-1R/CD52, IGF-1R/CD55, IGF-1R/CD58, IGF-1R/CD59, IGF-1R/CD82, IGF-1R/CD95, IGF-1R/CD97, IGF-1R/CD122, IGF-1R/CD124, IGF-1R/CD132, IGF-1R/CDw137, FAP-alpha/CD11a, FAP-alpha/CD15, FAP-alpha/CD18, FAP-alpha/CD29, FAP-alpha/CD39, FAP-alpha/CD45, FAP-alpha/CD48, FAP-alpha/CD52, FAP-alpha/CD55, FAP-alpha/CD58, FAP-alpha/CD59, FAP-alpha/CD82, FAP-alphalCD95, FAP-alpha/CD97, FAP-alpha/CD122, FAP-alpha/CD124, FAP-alpha/CD132, FAP-alpha/CDw137, CD19/CD11a, CD19/CD15, CD19/CD18, CD19/CD29, CD19/CD39, CD19/CD45, CD19/CD48, CD19/CD52, CD19/CD55, CD19/CD58, CD19/CD59, CD19/CD82, CD19/CD95, CD19/CD97, CD19/CD122, CD19/CD124, CD19/CD132, CD19/CDw137, CD52/CD11a, CD52/CD15, CD52/CD18, CD52/CD29, CD52/CD39, CD52/CD45, CD52/CD48, CD52/CD52, CD52/CD55, CD52/CD58, CD52/CD59, CD52/CD82, CD52/CD95, CD52/CD97, CD52/CD122, CD52/CD124, CD52/CD132, CD52/CDw137, GD2/CD11a, GD2/CD15, GD2/CD18, GD2/CD29, GD2/CD39, GD2/CD45, GD2/CD48, GD2/CD52, GD2/CD55, GD2/CD58, GD2/CD59, GD2/CD82, GD2/CD95, GD2/CD97, GD2/CD122, GD2/CD124, GD2/CD132, GD2/CDw137, CEA/CD11a, CEA/CD15, CEA/CD18, CEA/CD29, CEA/CD39, CEA/CD45, CEA/CD48, CEA/CD52, CEA/CD55, CEA/CD58, CEA/CD59, CEA/CD82, CEA/CD95, CEA/CD97, CEA/CD122, CEA/CD124, CEA/CD132, CEA/CDw137, FR/CD11a, FR/CD15, FR/CD18, FR/CD29, FR/CD39, FR/CD45, FR/CD48, FR/CD52, FR/CD55, FR/CD58, FR/CD59, FR/CD82, FR/CD95, FR/CD97, FR/CD122, FR/CD124, FR/CD132, FR/CDw137, proteoglycans/CD11a, proteoglycans/CD15, proteoglycans/CD18, proteoglycans/CD29, proteoglycans/CD39, proteoglycans/CD45, proteoglycans/CD48, proteoglycans/CD52, proteoglycans/CD55, proteoglycans/CD58, proteoglycans/CD59, proteoglycans/CD82, proteoglycans/CD95, proteoglycans/CD97, proteoglycans/CD122, proteoglycans/CD124, proteoglycans/CD132, proteoglycans/CDw137, G250/CD11a, G250/CD15, G250/CD18, G250/CD29, G250/CD39, G250/CD45, G250/CD48, G250/CD52, G250/CD55, G250/CD58, G250/CD59, G250/CD82, G250/CD95, G250/CD97, G250/CD122, G250/CD124, G250/CD132, G250/CDw137, GC182/CD11a, GC182/CD15, GC182/CD18, GC182/CD29, GC182/CD39, GC182/CD45, GC182/CD48, GC182/CD52, GC182/CD55, GC182/CD58, GC182/CD59, GC182/CD82, GC182/CD95, GC182/CD97, GC182/CD122, GC182/CD124, GC182/CD132, GC182/CDw137, GT468/CD11a, GT468/CD15, GT468/CD18, GT468/CD29, GT468/CD39, GT468/CD45, GT468/CD48, GT468/CD52, GT468/CD55, GT468/CD58, GT468/CD59, GT468/CD82, GT468/CD95, GT468/CD97, GT468/CD122, GT468/CD124, GT468/CD132, GT468/CDw137, GT512/CD11a, GT512/CD15, GT512/CD18, GT512/CD29, GT512/CD39, GT512/CD45, GT512/CD48, GT512/CD52, GT512/CD55, GT512/CD58, GT512/CD59, GT512/CD82, GT512/CD95, GT512/CD97, GT512/CD122, GT512/CD124, GT512/CD132 and GT512/CDw137.

More preferably, the trifunctional bispecific antibody of the present invention is directed against two antigens, wherein such two antigens combination is selected from a group consisting of EpCAM/CD11a, EpCAM/CD18, EpCAM/CD45, EpCAM/CD52, EpCAM/CD82, EpCAM/CD97, Her2neu/CD11a, Her2neu/CD18, Her2neu/CD45, Her2neu/CD52, Her2neu/CD82, Her2neu/CD97, MUC1/CD11a, MUC1/CD18, MUC1/CD45, MUC1/CD52, MUC1/CD82, MUC1/CD97, EGFR/CD11a, EGFR/CD18, EGFR/CD45, EGFR/CD52, EGFR/CD82, EGFR/CD97, EphA2/CD11a, EphA2/CD18, EphA2/CD45, EphA2/CD52, EphA2/CD82, EphA2/CD97, GD2/CD11a, GD2/CD18, GD2/CD45, GD2/CD52, GD2/CD82, GD2/CD97, CD20/CD11a, CD20/CD18, CD20/CD82, and CD20/CD97.

Preferred antibodies are heterologous trifunctional bispecific antibodies, preferably monoclonal, selected from one or more of the following combinations of isotypes:
- rat-IgG2b/mouse-IgG2a,
- rat-IgG2b/mouse-IgG2b,
- rat-IgG2b/mouse-IgG3;
- rat-IgG2b/human-IgG1,
- rat-IgG2b/human-IgG2
- rat-IgG2b/human-IgG3 [oriental allotype G3m(st)=binding to protein A], rat-IgG2b/human-IgG4;
- rat-IgG2b/rat-IgG2c;
- mouse-IgG2a/human-IgG3 [caucasian allotypes G3m(b+g)=no binding to protein A, in the following indicated as *]
- mouse-IgG2a/mouse-[VH-CH1, VL-CL]-human-IgG 1-[hinge]-human-IgG3*-[CH2-CH3]
- mouse-IgG2a/rat-[VH-CH1, VL-CL]-human-IgG 1-[hinge]-human-IgG3*-[CH2-CH3]
- mouse-IgG2a/human-[VH-CH1, VL-CL]-human-IgG1-[hinge]-human-IgG3*-[CH2-CH3]
- mouse-[VH-CH1, VL-CL]-human-IgG1/rat-[VH-CH1, VL-CL]-human-IgG1-[hinge]-human-IgG3*-[CH2-CH3]
- mouse-[VH-CH1, VL-CL]-human-IgG4/rat-[VH-CH1, VL-CL]-human-IgG4-[hinge]-human-IgG4 [N-terminal region of CH2]-human-IgG3*[C-terminal region of CH2:>aa position 251]-human-IgG3*[CH3]
- at-IgG2b/mouse-[VH-CH1, VL-CL]-human-IgG1-[hinge-CH2-CH3]
- rat-IgG2b/mouse-[VH-CH1, VL-CL]-human-IgG2-[hinge-CH2-CH3]
- rat-IgG2b/mouse-[VH-CH1, VL-CL]-human-IgG3-[hinge-CH2-CH3, oriental allotype]
- rat-IgG2b/mouse-[VH-CH1, VL-CL]-human-IgG4-[hinge-CH2-CH3]
- human-IgG1/human-[VH-CH1, VL-CL]-human-IgG 1-[hinge]-human-IgG3*-[CH2-CH3]
- human-IgG1/rat-[VH-CH1, VL-CL]-human-IgG1-[hinge]-human-IgG4 [N-terminal region of CH2]-human-IgG3*[C-terminal region of CH2:>aa position 251]-human-IgG3*[CH3]
- human-IgG1/mouse-[VH-CH1, VL-CL]-human-IgG 1-[hinge]-human-IgG4 [N-terminal region of CH2]-human-IgG3*[C-terminal region of CH2:>aa position 251]-human-IgG3*[CH3]
- human-IgG1/rat-[VH-CH1, VL-CL]-human-IgG1-[hinge]-human-IgG2 [N-terminal region of CH2]-human-IgG3*[C-terminal region of CH2:>aa position 251]-human-IgG3*[CH3]
   human-IgG1/mouse-[VH-CH1, VL-CL]-human-IgG1-[hinge]-human-IgG2 [N-terminal region of CH2]-human-IgG3*[C-terminal region of CH2:>aa position 251]-human-IgG3*[CH3]
- human-IgG1/rat-[VH-CH1, VL-CL]-human-IgG1-[hinge]-human-IgG3*-[CH2-CH3]
- human-IgG1/mouse-[VH-CH1, VL-CL]-human-IgG1-[hinge]-human-IgG3*-[CH2-CH3]
- human-IgG2/human-[VH-CH1, VL-CL]-human-IgG2-[hinge]-human-IgG3*-[CH2-CH3]
- human-IgG4/human-[VH-CH1, VL-CL]-human-IgG4-[hinge]-human-IgG3*-[CH2-CH3]
- human-IgG4/human-[VH-CH1, VL-CL]-human-IgG4-[hinge]-human-IgG4 [N-terminal region of CH2]-human-IgG3*[C-terminal region of CH2:>aa position 251]-human-IgG3*[CH3]
- mouse-IgG2b/rat-[VH-CH1, VL-CL]-human-IgG 1-[hinge]-human-IgG3*-[CH2-CH3]
- mouse-IgG2b/human-[VH-CH1, VL-CL]-human-IgG 1-[hinge]-human-IgG3*-[CH2-CH3]
- mouse-IgG2b/mouse-[VH-CH1, VL-CL]-human-IgG 1-[hinge]-human-IgG3*-[CH2-CH3]

The trifunctional bispecific antibody with monovalent binding specificities used in one particularly preferred embodiment by the present invention has the following properties:
a) binding to a pan-leukocyte antigen, which is selected from at least one member of the group consisting of CD11a, CD15, CD18, CD29, CD39, CD45, CD48, CD52, CD55, CD58, CD59, CD82, CD95, CD97, CD122, CD124, CD132 and CDw137;
b) binding to a tumor-associated antigen on a tumor cell;
c) binding via its Fc-portion to an Fc-receptor positive cell;
the trifunctional bispecific antibody is further preferably selected of a group of antibodies with the following isotype combinations:
rat-IgG2b/mouse-IgG2a,
rat-IgG2b/mouse-IgG2b,
rat-IgG2b/human-IgG1,
mouse-[VH-CH1; VL-CL]-human-IgG1/rat-[VH-CH1, VL-CL]-human-IgG1-[hinge]-human-IgG3*-[CH2-CH3]
[* = Caucasian allotypes G3m(b+g) = no binding to protein A].

Specifically preferred is an antibody, preferably a trifunctional bispecific antibody and/or a scaffold protein directed against EpCAM and CD52 with the isotype combination rat-IgG2b/mouse-IgG2a. A preferred example for said trifunctional bispecific antibody is anti-CD52 x anti-EpCAM antibody. Preferably said antibody is monoclonal.

Preferably, the antibodies according to the invention are monoclonal, chimeric, recombinant, synthetic, semi-synthetic, or chemically modified intact antibodies having for example Fv, Fab, scFv, or F (ab)2 fragments.

In the method of the present invention also antibodies or derivatives or fragments of human origin can be used, or antibodies modified to be suitable for the use in humans (so-called "humanized antibodies") (see for example Shalaby et al., J. Exp. Med. 175 (1992), 217; Mocikat et al., Transplantation 57 (1994), 405).

The preparation of the different types of antibodies and antibody fragments mentioned above is obvious to the skilled artisan. The preparation of monoclonal antibodies preferably of mammalian origin, e.g. of human, rat, mouse, rabbit, or goat, can be performed using conventional methods for example as those described in Köhler and Milstein (Nature 256 (1975), 495), in Harlow and Lane (Antibodies, A Laboratory Manual (1988), Cold Spring Harbor) or in Galfré (Meth. Enzymol. 73 (1981), 3).

It is further possible to prepare the antibodies described by means of recombinant DNA technology according to techniques obvious to the skilled artisan (see Kurucz et al., J. Immunol. 154 (1995), 4576; Hollinger et al., Proc. Natl. Acad. Sci. USA 90 (1993), 6444).

The preparation of antibodies having two different specificities, the so-called bispecific antibodies, can be performed for example using recombinant DNA technology but also by the so-called hybrid hybridoma fusion technique (see for example Milstein et al., Nature 305 (1983), 537). This technique comprises fusing hybridoma cell lines each producing antibodies having one of the desired specificities and identifying and isolating recombinant cell lines producing antibodies having both specificities.

The problem forming the basis of the invention can be overcome by using in preferred embodiments either trifunctional bispecific or trispecific trifunctional antibodies if they exhibit the properties and effects as described herein. The invention is particularly described by the way of trifunctional bispecific antibodies. However, it is understood that it also covers the following trispecific antibodies exhibiting similar effects. Although above, the terms "antibody" or "scaffold protein" may refer to trifunctional bispecific antibody, it is understood that it can also cover the following trispecific antibodies exhibiting similar effects.

The preparation of antibodies exhibiting three specificities, so-called trispecific antibodies, also suitable to solve the problem of the invention may for example be carried out by coupling a third antigen binding site having an additional specificity, e.g. in the form of "single chain variable fragments" (scFv) to one of the IgG heavy chains of a bispecific antibody. Further, recombinant technology can be used, e.g. vector-based methods for protein synthesis or oligonucleotide synthesis.

Analogously, trispecific F(ab)2 constructs may be prepared by replacing the CH2-CH3 regions of the heavy chain of one specificity of a bispecific antibody by an scFv having a third specificity, while the CH2-CH3 regions of the heavy chain having the other specificity can be removed for example by insertion of a stop codon (at the end of the "hinge" 5 region) into the coding gene, e.g by homologous recombination.

It is also possible to prepare trispecific scFv constructs wherein three VH-VL regions representing three different specificities are arranged in series.

Intact bispecific antibodies are composed of two antibody semi-molecules (each having a H and a L immunoglobulin chain) each representing a specificity, and additionally like normal antibodies having a Fc portion performing the well-known effector functions. They are preferably prepared using the quadroma technology. This method of preparation is exemplified in DE-A-44 19 399. For complete disclosure this document is incorporated in its entirety by reference also with respect to a definition of bispecific antibodies. It should be understood that other methods of preparation are also useful if they lead to the intact bispecific antibodies according to the above definition required according to the invention.

For example, intact bispecific antibodies may be produced in sufficient amounts using a newly developed method of preparation (Lindhofer et al., J. Immunology, 155:219 (1995)). The combination of two bispecific antibodies directed against two different tumor-associated antigens (e.g. c-erb-B2, EpCAM, such as GA-733-2=C215) on the mammary carcinoma cells minimizes the risk that tumor cells expressing only one of the antigens remain unidentified.

In accordance with the method of the present invention, the antibodies or the scaffold proteins recognizing the tumor-associated antigens on a tumor cell are not bound to or by any means like magnetic particles or magnetic beads which might have been used in the art as a tool in order to remove associates which are formed via the binding of said antibodies to said tumor cells and optionally also immune cells. Further, the prior art describes the labeling of the tumor cells or the antibodies with e.g. chromogenic substances like fluorochromes followed by cell sorting like flow cytometry. Contrary thereto, the principle of the invention lies in the formation of three dimensional networks (associates) as described above of a size sufficient to be retained by filters or to be separated by centrifugal forces and the direct removal of said networks by mechanical means like filtration or centrifugation or a combination thereof. The size of the associates formed by interaction with the antibodies is in a magnitude to be removable by filtration or centrifugation. Embodiments with magnetic beads or similar tools which bind to the antibodies or the tumor cells as indirect auxiliary means to remove the associates between the antibodies and the tumor cells can be combined with the present invention. Also separation methods like flow cytometric methods wherein tumor cells are separated from normal cells can also be combined with the present invention.

The terms "associates", "multi-cellular complexes" and "aggregates" are used interchangeably and always define a three-dimensional network between antibodies and/or scaffold proteins, tumor cells and/or immune cells and/or further tumor cells in order to form cross-linked tumor cells which are removable by centrifugation or filtration or a combination thereof. Said associates are specifically comprised of antibodies, tumor cells and immune cells which are leukocytes and/or Fc-receptor positive cells.

The time period used in the present invention is 10 to 240 minutes, preferably 10 to 180 minutes, to obtain the intra-operatively salvaged blood containing said cell aggregates. In the present invention, a contact period is also preferably between 20 and 210 minutes, more preferably between 30 and 180 minutes and even more preferably between 30 and 120 minutes. In this regard, it is found that said time period is surprisingly critical for achieving the desired effects. When said time period is less than 10 minutes, the formation of the aggregates is not sufficient. When the time period is more than 180 minutes, immunologic processes could be initiated by bound antibodies to immune cells. Particularly when the time period is more than 240 minutes, the initiation of unwanted immunological processes could be observed. The temperature is preferably room temperature which might range from 19°C to 25°C, preferably about 21°C.

The blood products obtained by using the method of the invention are - according to a preferred embodiment - then re-administered into the patient from whom the starting blood sample was obtained, which would not cause severe side effects.

### Contacting the antibody and/or the scaffold protein with IBS

Preferably, provided that any of the antibodies is not able to bind to immune cells but only to identical or different tumor-associated antigens, it is of crucial importance that there is a cross-linkage between at least two different tumor cells in order to obtain associates between the antibody and at least two tumor cells.

The terms "associates", "multi-cellular complexes" and "aggregates" are used interchangeably and always define a three-dimensional network between antibodies, scaffold proteins, tumor cells and/or immune cells and/or further tumor cells in order to form cross-linked tumor cells which are removable by centrifugation or filtration or a combination thereof. Said associates are specifically comprised of antibodies, tumor cells and leukocyte cells.

Preferably, the at least one antibody is contacted with said intra-operatively obtained blood salvage for a time period which is sufficient to cross-link tumor cells and optionally immune cells and/or other tumor cells in order to obtain associates. Preferably, said tumor cells and said immune cells are present in said intra-operatively obtained blood salvage. Said tumor cells can be specifically recognized by said at least one antibody or said at least one scaffold protein.

The time period which is necessary to achieve said cross-linkage can be determined by a person skilled in the art with routine methods. The time period used in the invention is 10 to 240 minutes, preferably 10 to 180 minutes. Preferably a contact period is between 10 and 118 minutes, more preferably between 20 and 90 minutes and even more preferably between 30 and 60 minutes. The temperature is preferably room temperature which might range from 19°C to 25°C, preferably about 21°C.

The amount of antibodies which have to be applied in order to obtain said associates comprising antibodies may be determined by a person skilled in the art by routine measurements. The amount of antibody and/or scaffold protein to be applied is generally dependent on the binding affinity of the antibody and/or scaffold protein to said tumor antigen. Preferably, further amounts are 1 µg to 20 µg per liter IBS, more preferably 1 µg to 10 µg per liter IBS, 1 µg to 5 µg per liter IBS or 1 µg to 2 µg per liter IBS.

Preferably at least two different antibodies and/or scaffold proteins are applied which tumor antigen specificity or pan-leukocyte surface marker specificity differs from each other provided that at least one of the antibodies falls within the limitations of claim 1.

### Removal of Tumor Cell and Antibody/Scaffold Protein Associates from IBS

The associates are removed finally from said intra-operatively obtained blood salvage (IBS) in order to obtain an IBS which is essentially free of tumor cells. Depletion of said cell aggregates is preferably achieved by centrifugation, by filtration or a combination thereof. In a preferred method, at least one centrifugation step and preferably further at least one filtration step are included.

The filtration is preferably a leukocyte reduction or depletion filtration wherein all leukocytes contaminated by said tumor cells are all or substantially removed while the erythrocytes pass the filter and are collected. The filters might be selected from screen filters with pore sizes of between 20 µm to 40 µm which retain the associates formed by the method of the invention and also for instance fibrin strands and clumps of dead cells. Erythrocytes which are about 8 µm in size may pass through the filters. Micro aggregate filters are preferably used for the reinfusion of shed autologous blood collected during or after surgery. In order to receive leukocyte reduced red blood cells, filtration through a standard blood administration filter with a pore size of between 170µm to 260 µm could be also used.

A review describing the cell saver devices including separation methods is Carless PA, Henry DA, Moxey AJ, O'Connell D, Brown T, Fergusson DA, Cell salvage for minimizing perioperative allogeneic blood transfusion, 2010, The Cochrane Collaboration, John Wiley & Sons, Ltd. which is fully incorporated by reference.

An example for a micro-aggregate blood filter in order to receive an erythrocyte containing fraction only is Pall SQ 40S Blood Transfusion Filter.

Preferably, centrifugation is performed by a density gradient centrifugation step followed by washing with e.g. physiological saline for a time and with a rotational speed sufficient to remove said tumor cells-comprising associates and to separate erythrocytes from said tumor cell associates and from leukocytes.

Preferably, filtration is performed generally for a time period sufficient to at least substantially remove said tumor cells-comprising associates and to separate erythrocytes from said tumor cell associates and from leukocytes.

In a preferred embodiment said concentrate of erythrocytes which is free or substantially free of contaminating tumor cells is used without addition of physiological saline.

Further filtration steps may be used in order to remove residual associates and/or leukocytes.

As the underlying leukocyte filtration technology is different from classical, micron-rated filtration procedures including clogging issues, this advanced tumor cell removal strategy appears to be feasible.

It is one of the benefits of the invention that the time, complexity, selectivity, intensity of manipulation involved for said removal of tumor cells is dramatically reduced by the invention compared to the depletion of tumor cells by destruction with antibodies or by removal with magnetic beads or irradiation or cell sorting methods making the removal of tumor cells feasible for the limited time span during tumor surgery.

Other advantages of the present invention include that tumor cells from the produced erythrocyte concentrate out of operational blood are remarkably reduced, and proinflammatory cytokines produced in the operational field by traumata through surgery is also significantly decreased. Moreover, residual trifunctional antibody can be reduced to uncritical amount in the final EC concentrate ready for reinfusion.

Preferably, the blood products obtained by using the method of the invention are then re-administered into the patient from whom the starting blood sample was obtained.

### Figures

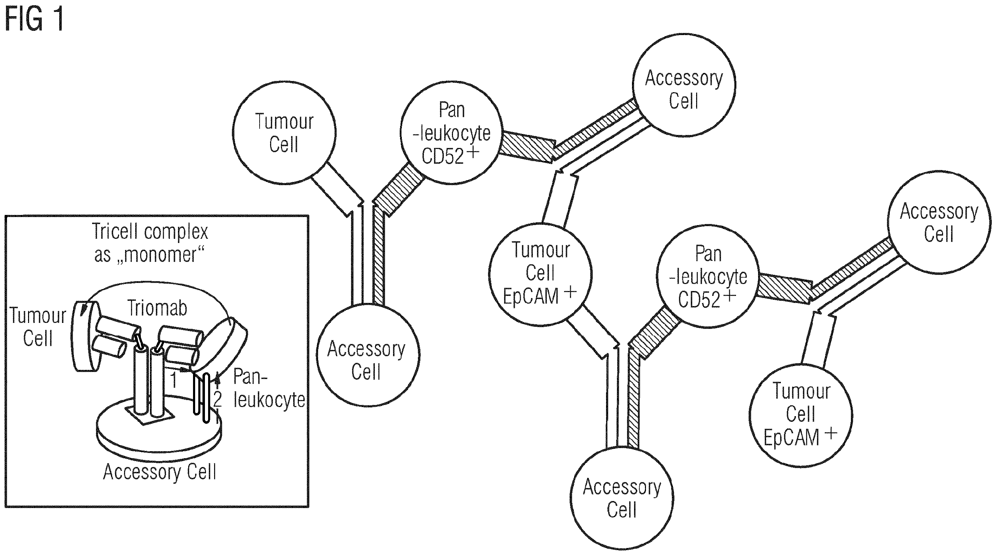

Figure 1: The principle of formation of large multicell complexes - induced by the trifunctional antibody as used in the present invention, e.g. anti-CD52 x anti-EpCAM antibody- is shown. Said complex formation allows tumor cell removal by intraoperative blood sample device-specific processing steps (e.g. centrifugation and filtration)
Figure 2: Schematic drawings of representative antibody formats that have the capacity to form multicellular complexes consisting of tumor cells and peripheral blood leukocytes (i.e. immune cells). Drawings are taken from Jin P and Z Zhu. In: Bispecific Antibodies. Kontermann RE (ed.), Springer Heidelberg Dordrecht London New York, pp. 151-170 (2011) and have been slightly modified. Only those antibodies covered by present claim 1 fall under the invention; all other antibody formats are described only for information purposes.

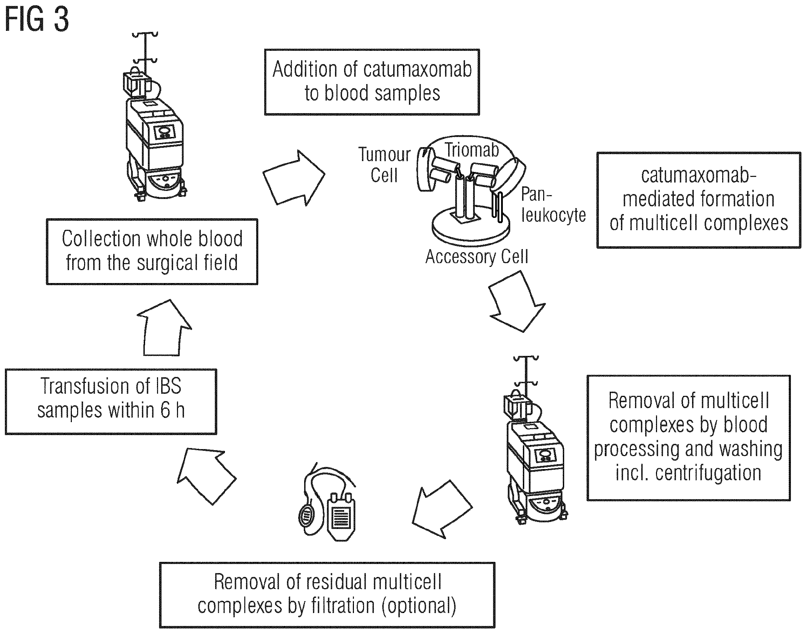

Figure 3: Exemplary embodiment of a method of the invention using a trifunctional bispecific antibody (BsAb) of the present invention, e.g. anti-CD52 x anti-EpCAM antibody.
Figure 4. Fed-batch culture Viable Cell Density (VCD) and Viability (VIA). F1-F4 - 1L-flask replicates.
Figure 5. AC chromatogram of the PGB0005 bispecific antibody purification.
Figure 6. PGB0005 bispecific antibody treated with DTT - light chains.
Figure 7. PGB0005 bispecific antibody treated with DTT - heavy chains.
Figure 8. PGB0005 bispecific antibody treated with PNGase F and DTT - heavy chains.
Figure 9. SDS-PAGE analysis of purified PGB0005 bispecific antibody. Lanes 1 and 12 - FB-blank, Lanes 2-6 reducing conditions, Lanes 7-12 - non-reducing conditions. Lanes 4 and 9 - PNGase F enzyme, Lanes 2 and 11 - non-deglycosylated PGB0005 bispecific antibody, Lanes 3, 5, 6, 7, 8 and 10 - deglycosylated PGB0005 bispecific antibody.
Figure 10. Fcγ-receptor (Fc gamma-receptor) binding activity of the PGB0005 bispecific antibody.
Figure 11. EpCAM binding activity of the PGB0005 bispecific antibody with: (a) all data points included, and (b) a single Hook effect data point removed.
Figure 12. CD52 binding activity of PGB0005 bispecific antibody with (a) solution buffer with pH=6.1, and (b) solution buffer with pH=7.4. BsAb FITC anti-mouse, BsAb FITC anti-rat - detection antibodies used in the assays.

### Examples

In the following, one exemplary example for carrying out the invention is described. A person with ordinary skill in the art following this approach will inevitably result in obtaining the claimed benefit. Following this example, various modifications can be performed without deviating from the invention, the scope of which is defined by the appended claims.

### Anti-EpCAM mediated removal of tumor cells through multicell complex depletion by centrifugation and /or filtration during IBS

Expression profiles of the transmembrane glycoprotein EpCAM classified by tissue microarray staining occurs in carcinomas of various tumors (Table. 3). This broad expression EpCAM pattern represents an interesting hallmark of carcinomas which may be not only considerable for therapeutical application but also for improved IBS protocols during cancer surgeries. Nevertheless, it should be noted that most soft-tissue tumors and all lymphomas were completely EpCAM negative.

**Table 3. Tissue microrarray: EpCAM expression profile on various tumor entities**

| **Tumor Entity** | **Number of samples (n)** | **Negative Expression (%)** | **Weak/Moderate Expression^{#} (%)** | **Strong Expression* (%)** |
|---|---|---|---|---|
| **Prostate¹** | | | | |
| | **414** | **1.9** | **10.9** | **87.2** |

| **Colon¹** | | | | |
|---|---|---|---|---|
| | **1186** | **0.3** | **1.9** | **97.7** |

| **Lung¹** | | | | |
|---|---|---|---|---|
| | **1287** | **13.5** | **22.5** | **63.9** |

| **Gastric¹** | | | | |
|---|---|---|---|---|
| | **473** | **2.5** | **6.8** | **90.7** |

| **Ovarian²** | | | | |
|---|---|---|---|---|
| | **272** | **2.6** | **12.1** | **85.3** |

| | | | | |
|---|---|---|---|---|
| ¹ Data taken from Went et al., Brit. J. Cancer 94: 128, 2006. ² Data taken from Spizzo et al., Gyneological Oncology 103: 483, 2006. ^{#} Score 1-4; EpCAM-expression was defined by calculating of total staining score as the product of a proportion score (0-4) and an intensity score (0-3). The proportion score described the estimated fraction of positive stained tumor cells (0, none; 1, < 10%; 2, 10-50%; 3, 50-80%; 4, >80%). The intensity score represented the estimated staining intensity (0, no staining; 1, weak; 2, moderate; 3, strong). * Score > 4; the total expression score ranged from 0 to 12, thus EpCAM overexpression was defined as a total score > 4. | | | | |

### Example 1

### 1. Obtaining an anti-CD52 x anti-EpCAM trifunctional bispecific antibody

In the backbone of a trifunctional bispecific antibody the anti-lymphocyte paratope was exchanged by the paratope of an anti-CD52 specific variable region binding to leukocytes. The sequence of the anti-CD52 specific variable regions (H+L IgG chains) is published by Crowe et al. (Clin Exp Immunol, vol. 87, 1992, Fig.2).

The antibody-coding genes of the heavy and light Ig-genes representing the anti-EpCAM half-antibody as well as the heavy and light genes representing the anti-CD52 half-antibody were transferred to a CHO cell line. The trifunctional antibody with the specificities anti-EpCAM and anti-CD52 secreted in the supernatant of cultured transfected CHO cells were purified with standard chromatographic methods.

To verify the functionality of the two binding arms, flow cytometric analysis of the binding to two different cell lines expressing either EpCAM or CD52 was performed.

### 2. Evaluating the binding of trifunctional bispecific anti-CD52 x anti-EpCAM antibody to different target antigens

Antibody binding was evaluated by FACS-analysis. 5 x 10⁵ target cells (CD52-positive Jurkat cells or EpCAM-positive HCT-8 cells were incubated at 2-8°C for 60 min at the indicated antibody concentrations. Target cells were resuspended in PBS buffer. Then, cells were washed two times and the antibody binding on cell was detected with fluorescence labeled (FITC) rat-anti-mouse IgG (Jurkat cells) or mouse-anti-rat IgG (HCT-8 cells) secondary detection antibodies (Dianova). Positively stained cells were evaluated using a FACS-Calibur cytometer (Becton Dickinson).

### 3. Evaluating the binding of trifunctional bispecific anti-CD52 x anti-EpCAM antibody to Fc-gamma receptors

Antibody binding was evaluated by FACS-analysis. 5 x 10⁵ target cells (Fc-gamma receptor positive THP-1 cells) were incubated at 2-8°C for 60 min at the indicated antibody concentrations. Target cells were resuspended in PBS buffer. Then, cells were washed two times and antibody binding on cell was detected with fluorescence labeled (FITC) F(ab)2 goat-anti-mouse IgG F(ab)2 secondary detection antibodies (Dianova). Positively stained cells were evaluated using a FACS-Calibur cytometer (Becton Dickinson).

### Example 2

### 1. Target molecule structure

PGB0005 bispecific antibody is a rat/mouse trifunctional antibody designed to target CD52 and EpCAM receptors with its variable regions. The original variable region of the rat half antibody has been exchanged to one targeting CD52, derived from Campath sequence described in literature. The mouse half antibody of Catumaxomab with a variable region targeting EpCAM was not modified. Gene sequences for both half antibodies were optimised for CHO expression.

### 2. Batch culture

### Method:

PGB0005 stable cell pool 5 was cultured in Excell-Advanced fed-batch medium in four 1L flasks with working volumes of 300ml, with initial seeding at cell density of 0.5×10⁶ cells/ml. Cell boost 7a and 7b feeding media were added daily at 3% and 0.3% of the working volume respectively, starting on the day 3 of the culture process.

### Results:

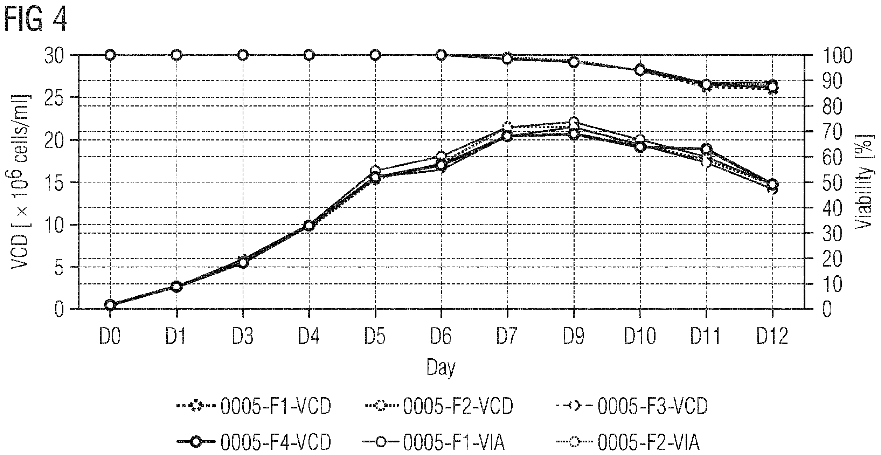

Maximum cell density of around 23×10⁶ cells/ml was achieved on day 9 of the culture process. Cell viability fell below 90% on day 11, and the culture was harvested on day 12 with average viable cell density over the four flasks of 23×10⁶ cells/ml and average viability of 86% (Fig. 4).

### 3. Purification

### Method:

1L of culture supernatant was purified by isocratic affinity chromatography. MabSelect Sure resin was used, with a column volume of 42ml. 0.1M Na-citrate was used as the elution buffer, with the following conditions for the separation of antibody fractions: pH5.8 for elution of the parental rat antibody, pH5.15 for the elution of the target PGB0005 bispecific antibody, and pH3.5 for the elution of the parental mouse antibody.

### Results:

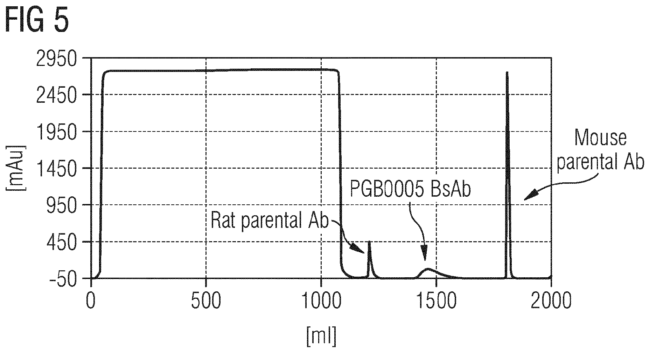

Target bispecific antibody was successfully separated from both parental antibodies (Fig. 5).

### 4. Quality analysis

Prior to initiating fed-batch culture production of the sample described, identity of protein produced by PGB0005 stable cell pool 5 was confirmed by LC-MS. Fed-batch produced purified PGB0005 bispecific antibody sample was analysed by SDS-PAGE and tested for Fcγ-receptor binding activity, EpCAM binding activity and CD52 binding activity.

### 4.1. LC-MS analysis of a purified stable cell pool 5-produced sample

### Method:

PGB0005 bispecific antibody sample produced in a flask culture and purified by AC chromatography was analysed by LC-MS. Prior to LC-MS analysis, sample underwent two different treatments: 1) denaturation followed by deglycosylation by PNGaseF treatment and DTT treatment, and 2) denaturation followed by DTT treatment. The samples were then injected into a BEH C4 column for separation and analysed by a Q-Tof Mass Spectrometer. Theoretical molecular weight information for the target protein is shown in the Table 1. Theoretical molecular weight information after post-translational modifications are shown in Tables 2 and 3.

**Table 4. Expected (non-reduced) molecular weight values for the PGB0005 bispecific antibody components.**

| Name | MW (Da) |
|---|---|
| Anti-CD52 light chain | 23175.9 |
| Anti-EpCAM light chain | 24424.8 * |
| Anti-CD52 heavy chain | 49850.4 |
| Anti-EpCAM heavy chain | 50021.8 |

**Table 5. Expected MW for anti-CD52 heavy chain with post-translational modifications. N-terminal pQ - N-terminal pyroglutamine modification, -Lys C-terminal - loss of C-terminal lysine, +GOF - GOF glycan.**

| Name | MW (Da) |
|---|---|
| Anti-CD52 heavy chain | 49850.4 |
| N-terminal pQ | -17 |
| -Lys C-terminal | -128.2 |
| +GOF | 1445.3 |
| Expected value | 51150.5 |

**Table 6. Expected MW for anti-EpCAM heavy chain with post-translational modifications. N-terminal pE - N-terminal pyroglutamic acid modification, -Lys C-terminal - loss of C-terminal lysine, +GOF - GOF glycan, +G1F - G1F glycan.**

| Name | MW (Da) |
|---|---|
| Anti-EpCAM heavy chain | 50021.8 |
| N-terminal pE | -18 |
| -Lys C-terminal | -128.2 |
| +GOF | 1445.3 |
| +G1f | 1607.5 |
| Expected value +GOF | 51320.9 |
| Expected value +G1F | 51483.1 |

**Table 7. Expected MW for anti-CD52 heavy chain with post-translational modifications but without N-glycan. N-terminal pQ - N-terminal pyroglutamine modification, -Lys C-terminal - loss of C-terminal lysine.**

| Name | MW (Da) |
|---|---|
| Anti-CD52 heavy chain | 49850.4 |
| N-terminal pQ | -17 |
| -Lys C-terminal | -128.2 |
| Expected MW | 49705.2 |

### Results:

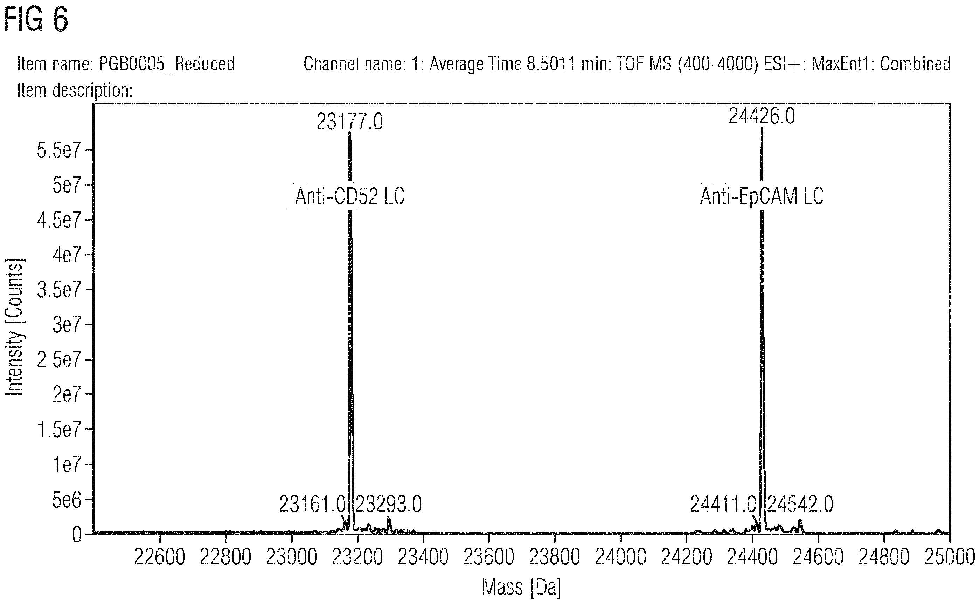

Anti-CD52 light chain and anti-EpCAM light chain were identified by MS in the non-PNGase F-treated sample (Fig. 6). Empirical MW obtained for anti-CD52 light chain was 23177.0Da, which corresponds well to the theoretical MV of 23175.9Da (Table 4). Empirical MW obtained for anti-CD52 light chain was 24426.0Da, which corresponds well to the theoretical MW of 24424.8Da (Table 1).

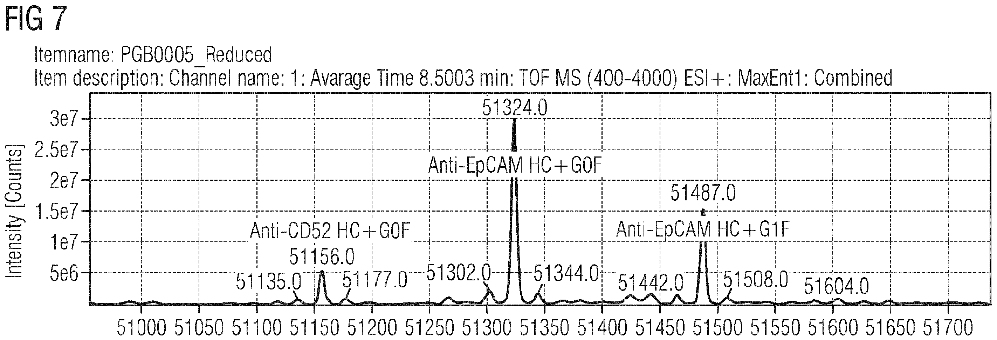

Anti-CD52 glycosylated heavy chain was identified, with MW of 51156.0Da (Fig. 7), which corresponds well with the theoretical value of 51150.5Da for the anti-CD52 heavy chain when post-translational modifications are taken into account (Table 5). Two MS peaks of 51156.0Da and 51487.0Da (Fig. 7) were identified, corresponding to expected MWs of anti-EpCAM heavy chain with GOF glycosylation (51320.9Da) and G1F glycosylation (51483.1Da) respectively (Table 6). The discrepancy between the expected and observed values of approximately 6Da for the GoF-glycosylated anti-EpCAM heavy chain, and of approximately 4Da for the G1F-glycosylated anti-EpCAM heavy chain might be explained by disulphide bond opening due to DTT treatment. As mass difference between anti-EpCAM heavy chain with GOF glycosylation and anti-CD52 chain with G1F glycosylation is less than 10Da, it is possible that their signals interfere with each other.

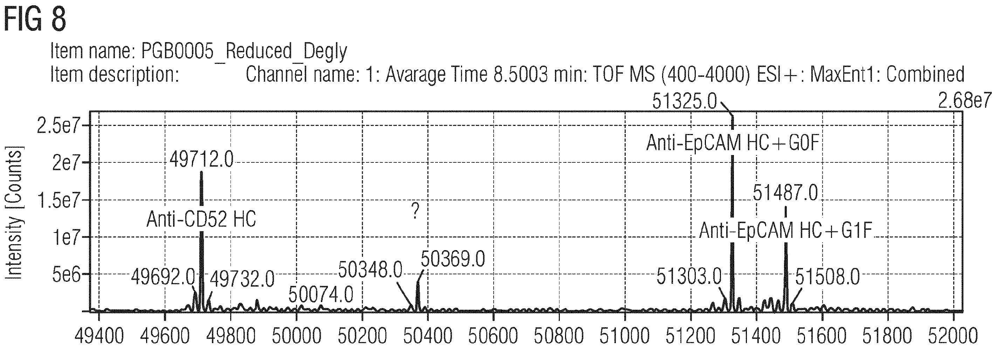

In Fig. 8, the observed 49712.0 Da peak was consistent with the expected MW of anti-CD52 heavy chain of 49705.2Da (Table 7). The approximately 6Da difference might be explained by disulfide bond opening due to DTT treatment.

### 4.2. SDS-PAGE analysis

### Method:

Purified PGB0005 sample was divided into two batches: 1) non-deglycosylated and 2) deglycosylated (treatment with PNGase F). The two batches were analysed by electrophoresis on SDS polyacrylamide gel under reducing and non-reducing conditions.

### Results:

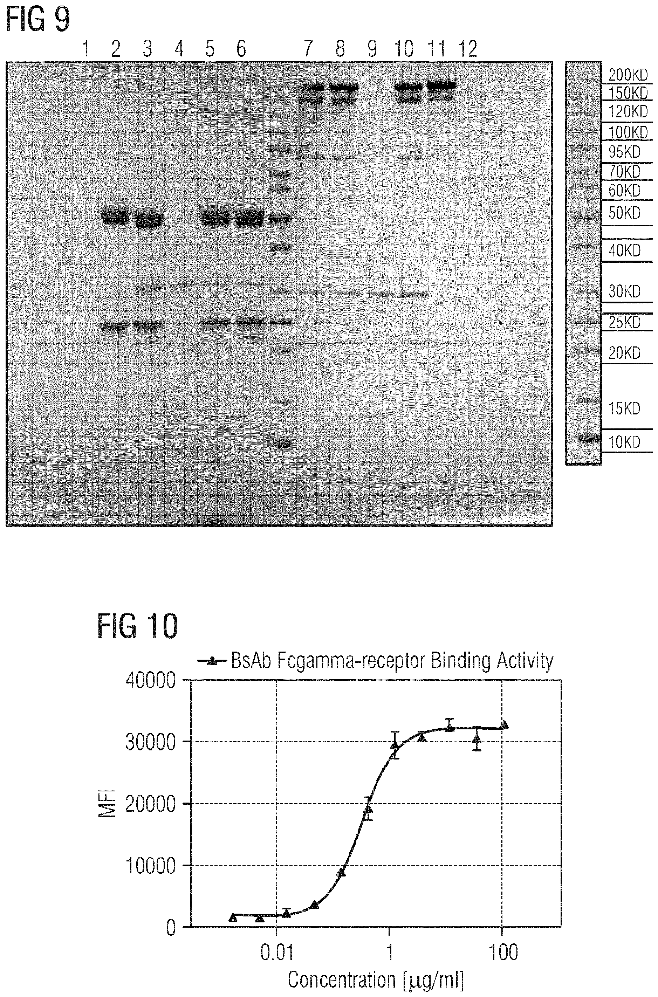

Two heavy chain bands at MW of approximately 50kD are seen in lane 2 (non-deglycosylated sample) and lanes 3, 5 and 6 (deglycosylated sample). Heavy chain bands in lane 2 (non-deglycosylated) have lower MW than those in lanes 3, 5 and 6 (deglycosylated) (Fig. 9).

Two light chain bands, MW approximately 25kD, are smeared together in lanes 2, 3, 5 and 6.

Two main bands, with MWs of approximately 150kD and 200kD respectively, are seen in lanes 7,8,10 and 11. The bands are of lower MW in lane 11 (non-deglycosylated) compared to lanes 7, 8 and 10 (deglycosylated).

Three additional bands, with MWs of approximately 120kD, 85kD and 22.5kD respectively, are seen in lanes 7, 8, 10 and 11.

### 4.3. Fcγ-receptor binding activity

### Method:

PGB0005 purified bispecific antibody sample was tested for Fcγ-receptor binding activity by flow cytometry (FACS).

THP-1 cell culture suspension was used at a density of 4×10⁶ cells/ml, at 50µl per well. Working sample concentrations used for the PGB0005 bispecific antibody were in the range of 0.0017-100 µg/ml, with 50µl per well.

Binding reaction proceeded for 1h at 2-8°C. PBS at pH7.4 was used as dilution buffer, and PBS at pH7.4 with 2%FBS was used as wash buffer.

FITC- goat anti-mouse F(ab')2 fragment specific was used as detection antibody. Antibody detection reaction proceeded for 30min at 2-8°C.

### Results:

PGB0005 bispecific antibody shows Fcγ-receptor binding activity (Fig. 10).

### 4.4. EpCAM binding activity

### Method:

PGB0005 purified bispecific antibody sample was tested for EpCAM binding activity by flow cytometry (FACS).

HCT-8 adherent cell culture was used at a density of 1.5×10⁶ cells/ml, at 100µl per well. Working sample concentrations used for the PGB0005 bispecific antibody were in the range of 0.0017-100 µg/ml, with 50µl per well.

Binding reaction proceeded for 1h at room temperature. PBS at pH7.4 with 2%FBS was used as dilution buffer, and also as cell cleaning solution.

FITC - mouse anti-rat (H+L) was used as detection antibody.

Antibody detection reaction proceeded for 30min at 2-8°C.

### Results:

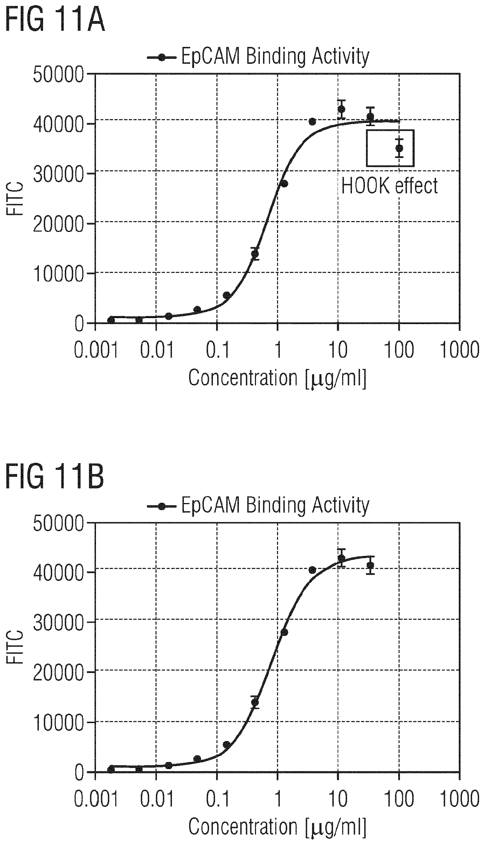

PGB0005 bispecific antibody shows EpCAM binding activity (Fig. 11). A single data point at a concentration of 100µg/ml was removed due to Hook effect.

### 4.5. CD52 binding activity

### Method:

Purified PGB0005 bispecific antibody samples dissolved in PBS buffer at two different pH values: of 7.4 and 6.1, were tested for CD52 binding by flow cytometry (FACS). Two different detection antibodies were used. Two replicate experiments were performed.

Ramos cell culture suspension was used at a density of 4×10⁶ cells/ml, at 50µl per well. Working sample concentrations used for the PGB0005 bispecific antibody samples are shown in the table below:

**Table 8. Working sample concentrations for the CD52 binding assay experiments.**

| Replicate | Buffer | pH | BsAb working concentration |
|---|---|---|---|
| 1 | PBS | pH7.4 | 0.0203-1196.9µg/mL |
| 1 | PBS | pH6.1 | 0.0172-1014.0µg/mL |
| 2 | PBS | pH7.4 | 0.0172-1017.2µg/mL |
| 2 | PBS | pH6.1 | 0.0147-867.6µg/mL |

Binding reaction proceeded for 1h at 2-8°C. Sample buffers (PBS at pH7.4 or pH6.1) were also used as dilution buffers, and PBS at pH7.4 with 2%FBS was used as wash buffer. FITC- mouse anti-rat (H+L) and FITC - rat anti-mouse (H+L) were used as detection antibodies.

Antibody detection reactions proceeded for 30min at 2-8°C.

### Results:

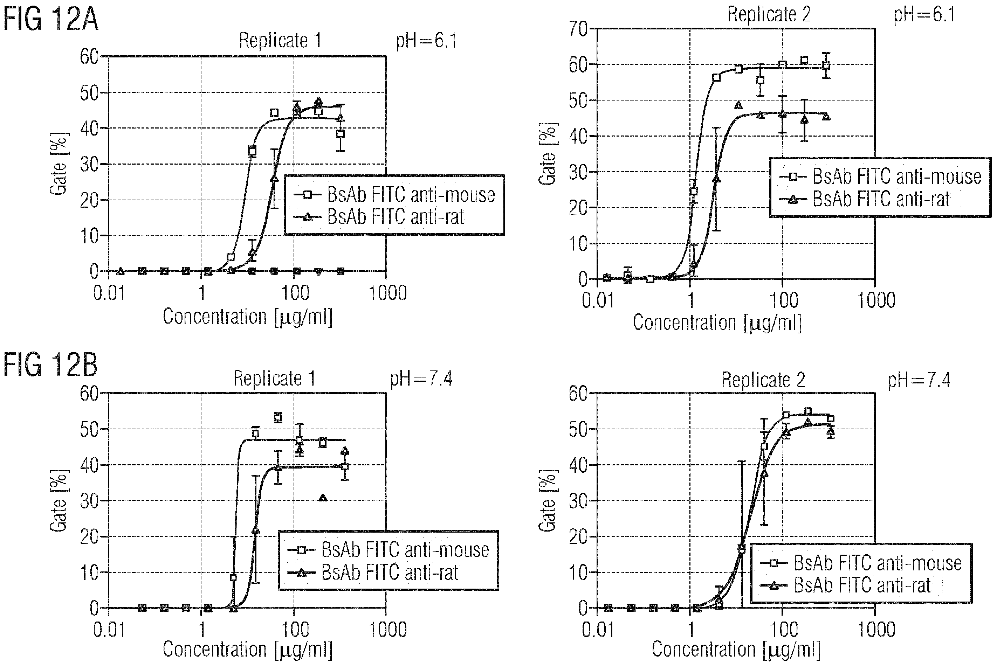

PGB0005 bispecific antibody shows CD52 binding activity at pH values of both 6.1 and 7.4 (Fig. 12). Rat-anti mouse detection antibody appears to bind stronger to the target bispecific antibody compared to the mouse anti-rat detection antibody. Results are consistent between two independent experiments.

### Summary

Both blood samples of cancer patients purified with the cell saver system and trifunctional bispecific anti-CD52 x anti-EpCAM antibody will show no or substantially substiantially no detectable remaining tumor cells (0) in the erythrocyte concentrates. Furthermore, cytokines which are secreted during surgery will be clearly decreased in the erythrocyte concentrate after the described procedure, revealing no safety issues for the patient. Importantly, residual anti-CD52 x anti-EpCAM antibody will not or substantially not be detectable in the produced erythrocyte concentrates even with a very sensitive ELISA method (detection limit: 250pg/ml) confirming the safety for potential patients receiving these purified autologous erythrocyte concentrates.

### Conclusions

Due to potential infection risks of tested and untested virus and other risks through allogeneic blood transfusions, increasing demands and economical needs, intraoperative blood salvage gains more interest in blood management programs of hospitals. Nevertheless, the use of intraoperative autotransfusion in major cancer surgeries has been limited due to the probability that tumor cells would be reinfused into the patient thus leading to augmented incidences of recurrence.

It has been found that the anti-CD52 x anti-EpCAM antibody with its binding capacity to carcinoma cells, leukocytes and accessory cells mediates the formation of multi-cellular complexes that could be simply removed by centrifugation and/or leukocyte filtration, conventional steps for leukocyte removal. The invention shows the potential anti-CD52 x anti-EpCAM antibody -mediated tumor cell/leukocyte removal capability during intraoperative blood salvage by using e.g. automatic devices like Cell Saver-5^{®} or CATS^{®}. The *in vitro* application of anti-CD52 x anti-EpCAM antibody represents an use as medical device and medical product, respectively, during the intraoperative blood salvage process in cancer surgery.

Starting with these experimentally supported data, it is evident for a person skilled in the art that this experimentally proved concept can also be transferred effectively to other monoclonal antibodies provided that they are able to interact with tumor cells and optionally with immune cells and/or other tumor cells in order to form aggregates or associates which can be removed by e.g. filtration or centrifugation. For these reasons, the present invention is not limited to trifunctional bispecific antibodies like anti-CD52 x anti-EpCAM antibody which recognizes the tumor-associated antigen EpCAM and the pan-leukocypte marker CD52 and the specific isotype combinations presented herein; having described herein the concept of the present invention, it can easily be transferred to other antibodies and tumor-associated antigens as described herein as long as they are able to recognize tumor-associated antigens and have the ability to form removable associates.

Taken together, the application of the method of the present invention, e.g. by using anti-CD52 x anti-EpCAM antibody to minimize the risk of residual autologous tumor cells would be a prerequisite to perform IBS during cancer surgery.

Compared with the conventional methods, the present invention provides a faster method with an improved efficiency to remove tumor cells from intraoperatively salvaged blood. The antibody disclosed in the present invention, particularly the trifunctional bispecific antibody, binds to leukocytes, tumor cells and Fc-gamma receptor positive accessory cells to form a three-dimensional network of antibodies and tumor cells and immune cells and further optionally or additionally other tumor cells. Said trifunctional bispecific antibody is able to bind with specific pan-leukocyte antigens, which allows the binding of various types of blood cells including T cells, almost up to 100% of the lymphocyte, with tumor cells. Therefore, compared with the other known methods, for example a method wherein the antibody mediates merely T cells to bind with tumor cells, the presently disclosed trifunctional bispecific antibody mediates a significantly increased amount of blood cells including leukocytes and lymphocytes, much higher than the amount of T cells, to interact with the tumor cells in the intraoperatively salvaged blood, and thereby the tumor cells can be more efficiently removed. In this regard, the present invention is particularly advantageous in the case when the cancer patient has a low amount of T cells in the peripheral blood, as the other known methods which depends on the binding of T cells with tumor cells may not be able to remove the tumor cells efficiently, and the present invention, however, has no such limitation, because said trifunctional bispecific antibody also binds with various other types of blood cells in addition to T cells.

During the incubation of antibody in the blood for removing tumor cells, cytokines could be released from immune cells and accumulated in erythrocyte concentrates under certain circumstances despite a washing step, which could potentially cause immune responses after the transfusion of such erythrocyte concentrates to the patient. In the other known methods for removing tumor cells from intraoperatively salvaged blood wherein T cells are mediated to bind with tumor cells, particularly when such binding is through T cell specific antigen CD3 or CD28, a large amount of cytokines could be released into blood in a short time, leading to a severe inflammatory response in the patient. In this regard, although the use of the trifunctional bispecific antibody of the present invention could theoretically also lead to a release of cytokines, the degree and the speed of such release should be significantly decreased, which therefore remarkably reduces the risk of occurrence of a severe inflammatory response caused by the released cytokines in the operation blood.

Further, as explained above, the antibody disclosed in the present invention, particularly the trifunctional bispecific antibody, mediates a large amount of blood cells including leukocytes and lymphocytes to interact with tumor cells. Thus, compared with the other known methods wherein for example only T cells are mediated to interact with tumor cells, the presently disclosed antibody has the potential to form larger associates and/or aggregates with blood cells. In addition, the presently disclosed antibody binds to leukocyte antigens which are not expressed on the surface of erythrocytes, which therefore avoids the binding with erythrocytes. Particularly the antibody of the present invention which binds with CD52 has been demonstrated to exhibit no binding with erythrocytes. Thus, the method of the present invention efficiently separate tumor cells from erythrocyte before reintroducing the purified blood to the patient.

### Blood Transfusion Filters for the Hospital Bedside/Laboratory (www.pall.com/medical_6595.asp)

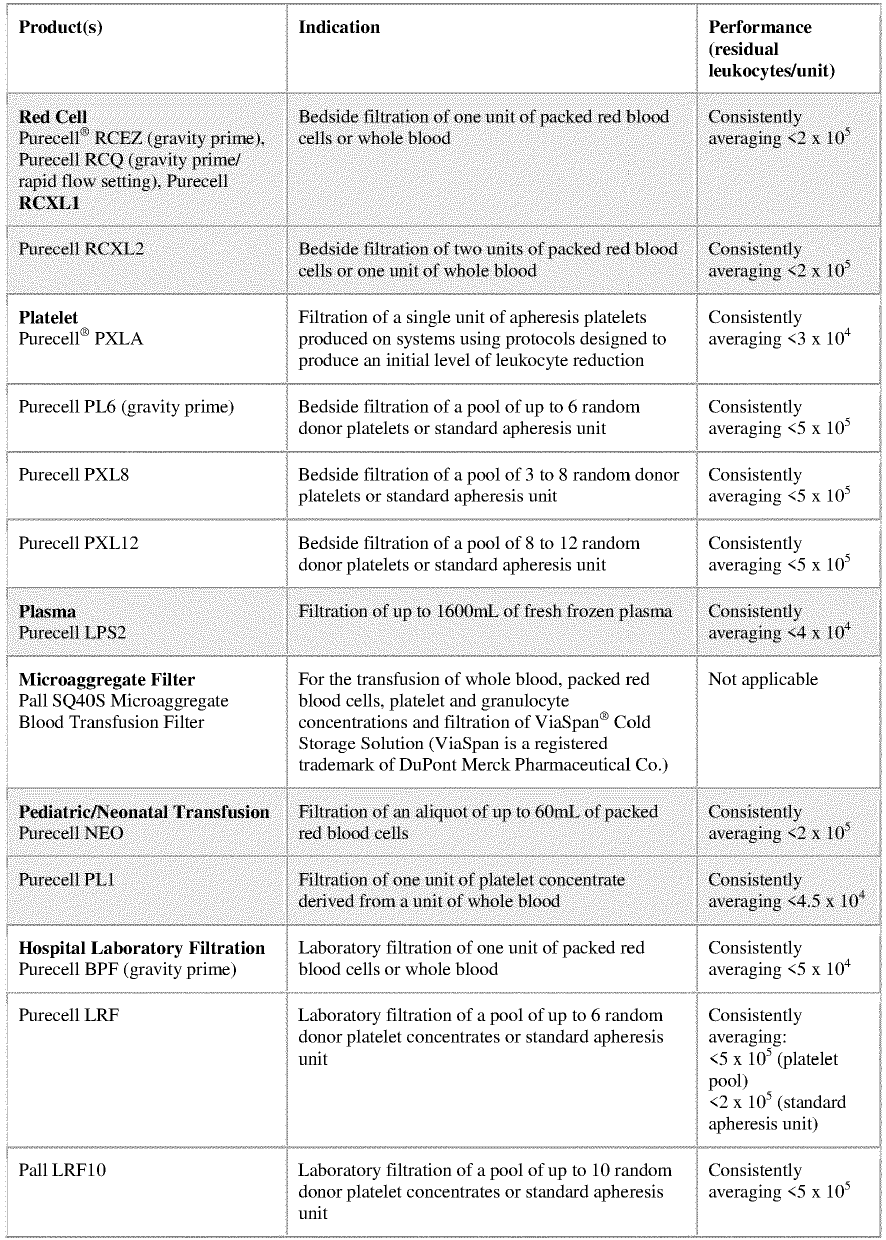

## Claims

1. An ex vivo method for removal of tumor cells from intraoperatively salvaged blood comprising the following steps:
- providing intraoperatively salvaged blood which may contain tumor cells outside the human body;
- contacting said intraoperatively salvaged blood with at least one trifunctional bispecific antibody,
wherein said at least one trifunctional bispecific antibody comprises the following properties:
a) binding to a pan-leukocyte antigen, which is selected from at least one member of the group consisting of CD11a, CD15, CD18, CD29, CD39, CD45, CD48, CD52, CD55, CD58, CD59, CD82, CD95, CD97, CD122, CD124, CD132, and CDw137;
b) binding to a tumor-associated antigen on a tumor cell;
c) binding via its Fc-portion to an Fc-receptor positive cell,
said at least one trifunctional bispecific antibody being capable of forming a 3-dimensional network at least with tumor cells and leukocyte cells contained in the blood,
wherein said trifunctional bispecific antibody is contacted with said intraoperatively salvaged blood for a time period of 10 - 240 minutes, preferably 10 - 180 minutes, which is sufficient to cross-link tumor cells comprising said tumor-associated antigen and leukocyte cells comprising said pan-leukocyte antigen, and/or other tumor cells in order to obtain associates and/or aggregates comprising said at least one trifunctional bispecific antibody, wherein said tumor cells, said other tumor cells and said leukocyte cells are potentially present in the intraoperatively salvaged blood, and wherein said tumor cells are specifically recognized by said at least one trifunctional bispecific antibody; and
- mechanically removing said associates and/or aggregates from said intra-operatively salvaged blood.

2. The ex vivo method according to claim 1, wherein said trifunctional bispecific antibody is selected of a group of antibodies with the following isotype combinations:
rat-IgG2b/mouse-IgG2a,
rat-IgG2b/mouse-IgG2b,
rat-IgG2b/human-IgG1,
mouse-[VH-CH1; VL-CL]-human-IgG1/rat-[VH-CH1, VL-CL]-human-IgG1-[hinge]-human-IgG3*-[CH2-CH3]
[* = Caucasian allotypes G3m(b+g) = no binding to protein A].

3. The ex vivo method according to claim 1 or 2, wherein said pan-leukocyte antigen is CD52.

4. The ex vivo method according to any one of claims 1 to 3, wherein at least two different antibodies are applied which tumor antigen specificity and/or pan-leukocyte antigen specificity differ from each other.

5. The ex vivo method according to any one of claims 1 to 4, wherein said associates are comprised of antibodies, tumor cells and leukocyte cells.

6. The ex vivo method according to any one of claims 1 to 5, wherein said one or more antibodies are applied in an amount of 1µg to 20µg, preferably 1 to 10µg or 1 to 5µg or 1 to 2µg per liter of intraoperatively salvaged blood.

7. The ex vivo method according to any one of claims 1 to 6, wherein said method comprises at least one of the following further steps:
a) mixing of the intraoperatively salvaged blood before addition of said at least one trifunctional bispecific antibody with at least one anticoagulating agent;
b) separating erythrocytes from said associates and/or aggregates and further blood components via centrifugation, preferably by density gradient centrifugation;
c) optionally filtration of said mixture
to remove potentially residual associates and/or aggregates and residual cell complexes; and
d) collecting the erythrocyte-containing fraction and further blood components in separate containers.

8. The ex vivo method according to any one of claims 1 to 7, wherein the incubation time of said at least one trifunctional bispecific antibody with said intraoperatively salvaged blood is between 10 and 90 minutes, further preferably between 20 and 60 minutes, optionally wherein said incubation is performed at a temperature of between 19 to 25°C, preferably at room temperature.

9. The ex vivo method according to any one of claims 1 to 8, wherein said removal of associates and/or aggregates is by centrifugation, filtration or a combination thereof.

10. The ex vivo method according to any one of claims 1 to 9, wherein leukocytes and/or tumor cell containing cell complexes are removed in a separate step using a leukocyte adsorption/depletion filter.

11. The ex vivo method according to any one of claims 1 to 10, wherein said tumor cells are from epithelial, hematological or neuroectodermal tumors.

12. The ex vivo method according to any one of claims 1 to 11, wherein additionaltrifunctional bispecific antibodies are administered which bind to a T cell, to a tumor-associated antigen on a tumor cell, and via its Fc-portion to an Fc-receptor positive cell.

13. The ex vivo method according to any one of claims 1 to 12, wherein said at least one trifunctional bispecific antibody comprises a property of binding to two different tumor-associated antigens.

14. Use of a bispecific trifunctional antibody, with the following properties:
a) binding to a pan-leukocyte antigen, which is selected from at least one member of the group consisting of CD11a, CD15, CD18, CD29, CD39, CD45, CD48, CD52, CD55, CD58, CD59, CD82, CD95, CD97, CD122, CD124, CD132, and CDw137;
b) binding to a tumor-associated antigen on a tumor cell;
c) binding via its Fc-portion to an Fc-receptor positive cell,
said antibody being capable of forming a 3-dimensional network with tumor cells and leukocyte cells contained in the blood,
in an ex vivo method according to one or more of the preceding claims 1 to 13, wherein preferably said at least one trifunctional bispecific antibody comprises a property of binding to two different tumor-associated antigens.

15. A trifunctional bispecific antibody for use in a method for treating cancer, wherein the method comprises the following steps:
- providing intra-operatively salvaged blood which may contain tumor cells;
- contacting said intra-operatively salvaged blood with said antibody, wherein said antibody comprises the following properties:
a) binding to a pan-leukocyte antigen, which is selected from at least one member of the group consisting of CDI11a, CD15, CD18, CD29, CD39, CD45, CD48, CD52, CD55, CD58, CD59, CD82, CD95, CD97, CD122, CD124, CD132 and CDw137; or
b) binding to a tumor-associated antigen on a tumor cell;
c) binding via its Fc-portion to an Fc-receptor positive cell,
said antibody being capable of forming a 3-dimensional network at least with tumor cells and leukocyte cells contained in the blood,
wherein said antibody is contacted with said intra-operatively salvaged blood for a time period of 10 - 240 minutes, preferably 10 - 180 minutes, which is sufficient to cross-link tumor cells comprising said tumor-associated antigen and leukocyte cells comprising said pan-leukocyte antigen, and/or other tumor cells in order to obtain associates and/or aggregates comprising said antibody, wherein said tumor cells, said other tumor cells and said leukocyte cells are potentially present in the intra-operatively salvaged blood, and wherein said tumor cells are specifically recognized by said antibody; and
- mechanically removing said associates and/or aggregates from said intra-operatively salvaged blood, wherein preferably said antibody comprises a property of binding to two different tumor-associated antigens.

## Patentansprüche

1. Ex-vivo-Verfahren zum Entfernen von Tumorzellen aus intraoperativ gewonnenem Blut, umfassend folgende Schritte:
- Bereitstellen von intraoperativ gewonnenem Blut, das Tumorzellen enthalten kann, außerhalb des menschlichen Körpers;
- Inkontaktbringen des intraoperativ gewonnenen Bluts mit mindestens einem trifunktionalen bispezifischen Antikörper,
wobei der mindestens eine trifunktionale bispezifische Antikörper die folgenden Eigenschaften umfasst:
a) Binden an ein Panleukozytenantigen, das ausgewählt ist aus mindestens einem Element der Gruppe bestehend aus CD11a, CD15, CD18, CD29, CD39, CD45, CD48, CD52, CD55, CD58, CD59, CD82, CD95, CD97, CD122, CD124, CD132 und Cdwl37;
b) Binden an ein tumorassoziiertes Antigen auf einer Tumorzelle;
c) Binden über seinen Fc-Teil an eine Fc-rezeptorpositive Zelle,
wobei der mindestens eine trifunktionale bispezifische Antikörper in der Lage ist, zumindest mit Tumorzellen und Leukozyten, die im Blut enthalten sind, ein dreidimensionales Netz zu bilden,
wobei der trifunktionale bispezifische Antikörper mit dem intraoperativ gewonnenen Blut über einen Zeitraum von 10-240 Minuten, vorzugsweise 10-180 Minuten in Kontakt gebracht wird, der für eine Vernetzung von Tumorzellen, die das tumorassoziierte Antigen umfassen, und Leukozyten, die das Panleukozytenantigen umfassen, und/oder anderer Tumorzellen ausreichend ist, damit so Verbände und/oder Aggregate erhalten werden, die den mindestens einen trifunktionalen bispezifischen Antikörper umfassen, wobei die Tumorzellen, die anderen Tumorzellen und die Leukozyten in dem intraoperativ gewonnenen Blut potenziell vorhanden sind, und wobei die Tumorzellen von dem mindestens einen trifunktionalen bispezifischen Antikörper spezifisch erkannt werden; und
- mechanisches Entfernen der Verbände und/oder Aggregate aus dem intraoperativ gewonnenen Blut.

2. Ex-vivo-Verfahren nach Anspruch 1, wobei der trifunktionale bispezifische Antikörper ausgewählt ist aus einer Gruppe von Antikörpern mit den folgenden Isotyp-Kombinationen:
IgG2b Ratte/IgG2a Maus,
IgG2b Ratte/IgG2b Maus,
IgG2b Ratte/IgG1 Mensch,
[VH-CH1; VL-CL] Maus - IgG1 Mensch/[VH-CH1, VL-CL] Ratte - IgG1 Mensch - [Gelenkregion] - IgG3* Mensch -[CH2-CH3]
[* = kaukasische Allotypen G3m(b+g) = keine Bindung an Protein A].

3. Ex-vivo-Verfahren nach Anspruch 1 oder 2, wobei das Panleukozytenantigen CD52 ist.

4. Ex-vivo-Verfahren nach einem der Ansprüche 1 bis 3, wobei mindestens zwei verschiedene Antikörper verwendet werden, deren Tumorantigenspezifität und/oder Panleukozyteantigenspezifität sich voneinander unterscheiden.

5. Ex-vivo-Verfahren nach einem der Ansprüche 1 bis 4, wobei die Verbände Antikörper, Tumorzellen und Leukozyten umfassen.

6. Ex-vivo-Verfahren nach einem der Ansprüche 1 bis 5, wobei der eine oder die mehreren Antikörper in einer Menge von 1 µg bis 20 µg, vorzugsweise 1 bis 10 µg oder 1 bis 5 µg oder 1 bis 2 µg pro Liter intraoperativ gewonnenem Blut verwendet werden.

7. Ex-vivo-Verfahren nach einem der Ansprüche 1 bis 6, wobei das Verfahren mindestens einen aus den folgenden weiteren Schritten umfasst:
a) Mischen des intraoperativ gewonnenen Bluts vor dem Zugeben des mindestens einen trifunktionalen bispezifischen Antikörpers mit mindestens einem Gerinnungshemmer;
b) Abtrennen von Erythrozyten aus den Verbänden und/oder Aggregaten und weiteren Blutbestandteilen mittels Zentrifugation, vorzugsweise mittels Dichtegradientenzentrifugation;
c) optional Filtern der Mischung zum Entfernen von möglicherweise verbleibenden Verbänden und/oder Aggregaten und verbleibenden Zellkomplexen; und
d) Sammeln der erythrozytenhaltigen Fraktion und weiteren Blutbestandteile in separaten Behältern.

8. Ex-vivo-Verfahren nach einem der Ansprüche 1 bis 7, wobei die Inkubationsdauer für den mindestens einen trifunktionalen bispezifischen Antikörper mit dem intraoperativ gewonnenen Blut zwischen 10 und 90 Minuten, weiter bevorzugt zwischen 20 und 60 Minuten beträgt, wobei optional die Inkubation bei einer Temperatur zwischen 19 und 25 °C, vorzugsweise bei Zimmertemperatur durchgeführt wird.

9. Ex-vivo-Verfahren nach einem der Ansprüche 1 bis 8, wobei das Entfernen von Verbänden und/oder Aggregaten durch Zentrifugation, Filtern oder eine Kombination daraus erfolgt.

10. Ex-vivo-Verfahren nach einem der Ansprüche 1 bis 9, wobei Leukozyten und/oder tumorzellhaltige Zellkomplexe in einem separaten Schritt unter Verwendung eines Leukozytenadsorptions-/-depletionsfilters entfernt werden.

11. Ex-vivo-Verfahren nach einem der Ansprüche 1 bis 10, wobei die Tumorzellen von epithelialen, hämatologischen oder neuroektodermalen Tumoren stammen.

12. Ex-vivo-Verfahren nach einem der Ansprüche 1 bis 11, wobei zusätzliche trifunktionale bispezifische Antikörper gegeben werden, die an eine T-Zelle, an ein tumorassoziiertes Antigen auf einer Tumorzelle und über ihren Fc-Teil an eine Fc-rezeptorpositive Zelle binden.

13. Ex-vivo-Verfahren nach einem der Ansprüche 1 bis 12, wobei der mindestens eine trifunktionale bispezifische Antikörper eine Eigenschaft aufweist, dass er an zwei verschiedene tumorassoziierte Antigene bindet.

14. Verwendung eines bispezifischen trifunktionalen Antikörpers mit den folgenden Eigenschaften:
a) Binden an ein Panleukozytenantigen, das ausgewählt ist aus mindestens einem Element der Gruppe bestehend aus CD11a, CD15, CD18, CD29, CD39, CD45, CD48, CD52, CD55, CD58, CD59, CD82, CD95, CD97, CD122, CD124, CD132 und Cdwl37;
b) Binden an ein tumorassoziiertes Antigen auf einer Tumorzelle;
c) Binden über seinen Fc-Teil an eine Fc-rezeptorpositive Zelle,
wobei der Antikörper in der Lage ist, mit Tumorzellen und Leukozyten, die im Blut enthalten sind, ein dreidimensionales Netz zu bilden,
in einem Ex-vivo-Verfahren nach einem oder mehreren der vorhergehenden Ansprüche 1 bis 13, wobei der mindestens eine trifunktionale bispezifische Antikörper vorzugsweise eine Eigenschaft aufweist, dass er an zwei verschiedene tumorassoziierte Antigene bindet.

15. Trifunktionaler bispezifischer Antikörper zur Verwendung in einem Verfahren zum Behandeln von Krebs, wobei das Verfahren die folgenden Schritte umfasst:
- Bereitstellen von intraoperativ gewonnenem Blut, das Tumorzellen enthalten kann;
- Inkontaktbringen des intraoperativ gewonnenem Bluts mit dem Antikörper, wobei der Antikörper die folgenden Eigenschaften umfasst:
a) Binden an ein Panleukozytenantigen, das ausgewählt ist aus mindestens einem Element der Gruppe bestehend aus CD11a, CD15, CD18, CD29, CD39, CD45, CD48, CD52, CD55, CD58, CD59, CD82, CD95, CD97, CD122, CD124, CD132 und Cdw137; oder
b) Binden an ein tumorassoziiertes Antigen auf einer Tumorzelle;
c) Binden über seinen Fc-Teil an eine Fc-rezeptorpositive Zelle,
wobei der Antikörper in der Lage ist, zumindest mit Tumorzellen und Leukozyten, die im Blut enthalten sind, ein dreidimensionales Netz zu bilden,
wobei der Antikörper mit dem intraoperativ gewonnenen Blut über einen Zeitraum von 10-240 Minuten, vorzugsweise 10-80 Minuten in Kontakt gebracht wird, der für eine Vernetzung von Tumorzellen, die das tumorassoziierte Antigen umfassen, und Leukozyten, die das Panleukozytenantigen umfassen, und/oder anderer Tumorzellen ausreichend ist, damit so Verbände und/oder Aggregate erhalten werden, die den Antikörper umfassen, wobei die Tumorzellen, die anderen Tumorzellen und die Leukozyten in dem intraoperativ gewonnenen Blut potenziell vorhanden sind, und wobei die Tumorzellen von dem Antikörper spezifisch erkannt werden; und
- mechanisches Entfernen der Verbände und/oder Aggregate aus dem intraoperativ gewonnenen Blut, wobei der Antikörper vorzugsweise eine Eigenschaft aufweist, dass er an zwei verschiedene tumorassoziierte Antigene bindet.

## Revendications

1. Procédé ex vivo pour l'élimination de cellules tumorales de sang récupéré en peropératoire, comprenant les étapes suivantes :
- fourniture de sang récupéré en peropératoire qui peut contenir des cellules tumorales à l'extérieur du corps humain ;
- mise en contact dudit sang récupéré en peropératoire avec au moins un anticorps bispécifique trifonctionnel,
dans lequel ledit au moins un anticorps bispécifique trifonctionnel comprend les propriétés suivantes :
a) liaison à un antigène pan-leucocytaire, qui est choisi parmi au moins un élément du groupe constitué de CD11a, CD15, CD18, CD29, CD39, CD45, CD48, CD52, CD55, CD58, CD59, CD82, CD95, CD97, CD122, CD124, CD132 et CDw137 ;
b) liaison à un antigène associé à une tumeur sur une cellule tumorale ;
c) liaison via sa portion Fc à une cellule positive au récepteur Fc,
ledit au moins un anticorps bispécifique trifonctionnel étant capable de former un réseau tridimensionnel au moins avec des cellules tumorales et des cellules leucocytaires contenues dans le sang,
dans lequel ledit anticorps bispécifique trifonctionnel est mis en contact avec ledit sang récupéré en peropératoire pendant une période de temps de 10 à 240 minutes, de préférence de 10 à 180 minutes, qui est suffisante pour réticuler des cellules tumorales comprenant ledit antigène associé à une tumeur et des cellules leucocytaires comprenant ledit antigène pan-leucocytaire et/ou d'autres cellules tumorales afin d'obtenir des produits associés et/ou des agrégats comprenant ledit au moins un anticorps bispécifique trifonctionnel, dans lequel lesdites cellules tumorales, lesdites autres cellules tumorales et lesdites cellules leucocytaires sont potentiellement présentes dans le sang récupéré en peropératoire, et dans lequel lesdites cellules tumorales sont spécifiquement reconnues par ledit au moins un anticorps bispécifique trifonctionnel ; et
- élimination mécanique desdits produits associés et/ou agrégats dudit sang récupéré en peropératoire.

2. Procédé ex vivo selon la revendication 1, dans lequel ledit anticorps bispécifique trifonctionnel est choisi parmi un groupe d'anticorps avec les combinaisons d'isotypes suivantes :
IgG2b de rat/IgG2a de souris,
IgG2b de rat/IgG2b de souris,
IgG2b de rat/IgG1 humain,
[VH-CH1 ; VL-CL] de souris-IgG1 humain/[VH-CH1, VL-CL] de rat-IgG1 humain-[articulation]-IgG3*-[CH2-CH3]
[* = allotypes caucasiens G3m(b+g) = pas de liaison à la protéine A].

3. Procédé ex vivo selon la revendication 1 ou 2, dans lequel ledit antigène pan-leucocytaire est CD52.

4. Procédé ex vivo selon l'une quelconque des revendications 1 à 3, dans lequel sont appliqués au moins deux anticorps différents, dont la spécificité d'antigène tumoral et/ou la spécificité d'antigène pan-leucocytaire diffèrent l'une de l'autre.

5. Procédé ex vivo selon l'une quelconque des revendications 1 à 4, dans lequel lesdits produits associés comprennent des anticorps, des cellules tumorales et des cellules leucocytaires.

6. Procédé ex vivo selon l'une quelconque des revendications 1 à 5, dans lequel ledit ou lesdits anticorps sont appliqués en une quantité de 1 µg à 20 µg, de préférence 1 à 10 µg ou de 1 à 5 µg ou de 1 à 2 µg par litre de sang récupéré en peropératoire.

7. Procédé ex vivo selon l'une quelconque des revendications 1 à 6, dans lequel ledit procédé comprend au moins une des étapes supplémentaires suivantes :
a) mélange du sang récupéré en peropératoire avant l'addition dudit au moins un anticorps bispécifique trifonctionnel avec au moins un agent anticoagulant ;
b) séparation des érythrocytes desdits produits associés et/ou agrégats et d'autres composants du sang par centrifugation, de préférence par centrifugation en gradient de densité ;
c) filtration facultative dudit mélange pour éliminer les produits associés potentiellement résiduels et/ou les agrégats et les complexes cellulaires résiduels ; et
d) collecte de la fraction contenant les érythrocytes et d'autres composants du sang dans des récipients séparés.

8. Procédé ex vivo selon l'une quelconque des revendications 1 à 7, dans lequel le temps d'incubation dudit au moins un anticorps bispécifique trifonctionnel avec ledit sang récupéré en peropératoire est compris entre 10 et 90 minutes, de manière plus préférée entre 20 et 60 minutes, facultativement dans lequel ladite incubation est réalisée à une température comprise entre 19 et 25 °C, de préférence à température ambiante.

9. Procédé ex vivo selon l'une quelconque des revendications 1 à 8, dans lequel ladite élimination de produits associés et/ou d'agrégats s'effectue par centrifugation, filtration ou une combinaison de celles-ci.

10. Procédé ex vivo selon l'une quelconque des revendications 1 à 9, dans lequel les leucocytes et/ou les complexes cellulaires contenant des cellules tumorales sont éliminés dans une étape séparée en utilisant un filtre d'adsorption/déplétion de leucocytes.

11. Procédé ex vivo selon l'une quelconque des revendications 1 à 10, dans lequel lesdites cellules tumorales proviennent de tumeurs épithéliales, hématologiques ou neuroectodermiques.

12. Procédé ex vivo selon l'une quelconque des revendications 1 à 11, dans lequel sont administrés des anticorps bispécifiques trifonctionnels supplémentaires qui se lient à une cellule T, à un antigène associé à une tumeur sur une cellule tumorale, et via sa portion Fc à une cellule positive au récepteur Fc.

13. Procédé ex vivo selon l'une quelconque des revendications 1 à 12, dans lequel ledit au moins un anticorps bispécifique trifonctionnel comprend une propriété de liaison à deux différents antigènes associés à une tumeur.

14. Utilisation d'un anticorps trifonctionnel bispécifique, avec les propriétés suivantes :
a) liaison à un antigène pan-leucocytaire, qui est choisi parmi au moins un élément du groupe constitué de CD11a, CD15, CD18, CD29, CD39, CD45, CD48, CD52, CD55, CD58, CD59, CD82, CD95, CD97, CD122, CD124, CD132 et CDw137 ;
b) liaison à un antigène associé à une tumeur sur une cellule tumorale ;
c) liaison via sa portion Fc à une cellule positive au récepteur Fc,
ledit anticorps étant capable de former un réseau tridimensionnel avec des cellules tumorales et des cellules leucocytaires contenues dans le sang,
dans un procédé ex vivo selon une ou plusieurs des revendications 1 à 13 précédentes, dans lequel ledit au moins un anticorps bispécifique trifonctionnel comprend de préférence une propriété de liaison à deux différents antigènes associés à une tumeur.

15. Anticorps bispécifique trifonctionnel destiné à être utilisé dans un procédé pour traiter un cancer, dans lequel le procédé comprend les étapes suivantes :
- fourniture de sang récupéré en peropératoire qui peut contenir des cellules tumorales ;
- mise en contact dudit sang récupéré en peropératoire avec ledit anticorps, dans lequel ledit anticorps présente les propriétés suivantes :
a) liaison à un antigène pan-leucocytaire, qui est choisi parmi au moins un élément du groupe constitué de CD11a, CD15, CD18, CD29, CD39, CD45, CD48, CD52, CD55, CD58, CD59, CD82, CD95, CD97, CD122, CD124, CD132 et CDw137 ; ou
b) liaison à un antigène associé à une tumeur sur une cellule tumorale ;
c) liaison via sa portion Fc à une cellule positive au récepteur Fc,
ledit anticorps étant capable de former un réseau tridimensionnel au moins avec des cellules tumorales et des cellules leucocytaires contenues dans le sang,
dans lequel ledit anticorps est mis en contact avec ledit sang récupéré en peropératoire pendant une période de temps de 10 à 240 minutes, de préférence de 10 à 180 minutes, qui est suffisante pour réticuler des cellules tumorales comprenant ledit antigène associé à une tumeur et des cellules leucocytaires comprenant ledit antigène pan-leucocytaire et/ou d'autres cellules tumorales afin d'obtenir des produits associés et/ou des agrégats comprenant ledit anticorps, dans lequel lesdites cellules tumorales, lesdites autres cellules tumorales et lesdites cellules leucocytaires sont potentiellement présentes dans le sang récupéré en peropératoire, et dans lequel lesdites cellules tumorales sont spécifiquement reconnues par ledit anticorps ; et
- élimination mécanique desdits produits associés et/ou agrégats dudit sang récupéré en peropératoire, dans lequel ledit anticorps comprend de préférence une propriété de liaison à deux différents antigènes associés à une tumeur.
